# EUROPEAN PATENT SPECIFICATION

(11) **EP 3 395 331 B1**
(45) Date of publication and mention of the grant of the patent: **21.08.2019**
(21) Application number: 17181421.3
(22) Date of filing: 14.07.2017
(51) Int. Cl.: A61P 7/00

(54) **PHARMACEUTICAL TABLET COMPOSITION COMPRISING ELTROMBOPAG OLAMINE**
PHARMAZEUTISCHE TABLETTENZUSAMMENSETZUNG UMFASSEND ELTROMBOPAGOLAMIN
COMPOSITION DE COMPRIMÉ PHARMACEUTIQUE COMPRENANT ELTROMBOPAG OLAMINE

(30) Priority: 26.04.2017 IN 201711014792
(43) Date of publication of application: 31.10.2018
(73) Proprietor: Alfred E. Tiefenbacher (GmbH & Co. KG), 22767 Hamburg (DE)
(72) Inventor: Staver, Ruslan, 22607 Hamburg (DE); Prathap, Vamshi Ramana, 502 319 Telangana (IN); Vadla, Hari Kiran Chary, 502319 Telangana (IN); Rallabandi, Bala Ramesha Chary, 502319 Telangana (IN); Schlehahn, Hendrik, 23843 Travenbrück (DE)
(74) Representative: Feldmann, Ute

(56) References cited:
- WO-A1-2008/136843
- WO-A2-2004/096154
- WO-A2-2015/029074

## Description

### Technical Field:

The present invention relates to a pharmaceutical tablet composition comprising eltrombopag olamine and one or more reducing sugars, a production process therefore, a pharmaceutical tablet composition comprising eltrombopag olamine and one or more reducing sugars obtainable by the production process, a use / method of use of the pharmaceutical tablet compositions in the treatment or prophylaxis of immune (idiopathic) thrombocytopenic purpura (ITP), thrombocytopenia and/or acquired severe aplastic anaemia (SAA).

### Background of the invention:

3'-(N'-[1-(3,4-dimethylphenyl)-3-methyl-5-oxo-1,5-dihydropyrazol-4-ylidene]hydrazino}-2'-hydroxy-biphenyl-3-carboxylic acid - 2-aminoethanol (1:2) (hereinafter and in the context of the present invention called eltrombopag olamine, CAS 496775-62-3) is a non-peptide hematopoietic receptor agonist, which mimics hematopoietic growth factors, including thrombopoietin (TPO), particularly enhancing platelet production and, thus, is particularly useful in the treatment of thrombocytopenia (see e.g. WO 03/098992 A2 and WO 2008/136843A1).

In Europe eltrombopag olamine is approved under the tradename Revolade® comprising eltrombopag olamine in an amount corresponding to 12.5 mg, 25 mg, 50 mg, and 75 mg eltrombopag free acid in tablet form. In USA eltrombopag olamine is approved under the tradename Promacta® comprising eltrombopag olamine in an amount corresponding to 12.5 mg, 25 mg, 50 mg, 75 mg and 100 mg eltrombopag free acid in tablet form. Revolade® tablets are packed in Alu-Alu blisters, whereas Promacta® tablets are packed in HDPE bottles.

In Europe, Revolade® is indicated for adult chronic immune (idiopathic) thrombocytopenic purpura (ITP) splenectomised patients who are refractory to other treatments (e.g. corticosteroids, immunoglobulins). Revolade® may be considered as second line treatment for adult non-splenectomised patients where surgery is contraindicated.

Revolade® is also indicated in adult patients with chronic hepatitis C virus (HCV) infection for the treatment of thrombocytopenia, where the degree of thrombocytopenia is the main factor preventing the initiation or limiting the ability to maintain optimal interferon-based therapy.

Revolade® is furthermore indicated in adult patients with acquired severe aplastic anaemia (SAA) who were either refractory to prior immunosuppressive therapy or heavily pretreated and are unsuitable for haematopoietic stem cell transplantation.

In WO 03/098992 A2 an eltrombopag tablet composition is disclosed in example 6 which comprises in addition to 8.45 mg eltrombopag olamine, furthermore 112 mg microcrystalline cellulose, 70 mg lactose, 8 mg sodium starch glycolate and 2 mg magnesium stearate.

It is, however, known, that eltrombopag olamine undergoes a Maillard reaction with respective pharmaceutically acceptable excipients, such as reducing sugars, e.g. lactose. It is known that eltrombopag olamine is degraded in presence of lactose and forms impurities, which can be measured in the pharmaceutical tablet composition. Mr. Kapsi, one of the inventors of WO 2008/136843 A1 provided in the corresponding US examination of US 2010/0040684 A1 a declaration (in the context of the present application called "Kapsi declaration") disclosing experimental stability data for the use of the lactose containing eltrombopag tablet composition of example 6 in WO 03/098992 A2 in comparison to a tablet formulation, which is free of reducing sugars using mannitol instead.

According to the Kapsi declaration, the eltrombopag olamine tablet formulation comprising lactose shows an *"increase in degradation products when compared to the mannitol based formulation (4 fold difference in degradation products at 3 months"* (see section Results on page 1). In section 5 on page 5 of the Kapsi declaration Mr. Kapsi followed that the *"experimental data described herein demonstrates that the formulation described as Example 6 in publication* WO 03/098992 A2 *leads to a tablet formulation with a four-fold higher level of degradation products at 3 months and in my experience leads to an unacceptable tablet formulation".*

In order to avoid unacceptable degradation of eltrombopag olamine, WO 2008/136843 A1 teaches to use diluents substantially free of reducing sugars, in particular using mannitol. The avoidance of reducing sugars as pharmaceutical excipient in an eltrombopag olamine pharmaceutical tablet is continued in WO 2012/121957 A1 and WO 2015/0121957 A2.

It would, however, be desirable to provide an alternative solution for a stable eltrombopag olamine pharmaceutical tablet composition, wherein the degradation level of eltrombopag olamine is negligible and the pharmaceutical tablet composition fulfills the regulatory stability criteria for pharmaceutical tablets. Furthermore, it would be desirable that the pharmaceutical tablet composition is more economic in costs per unit and/or allows tuning one or more pharmaceutical technological aspects during the production process.

### Brief description of the invention:

One or more problems of the present invention is/are solved by the subjects of the independent claims. Advantages (preferred embodiments) are set out in the detailed description hereinafter as well as in the dependent claims.

Accordingly, a first aspect of the present invention relates to a pharmaceutical tablet composition comprising eltrombopag olamine as active ingredient and one or more pharmaceutically acceptable excipients including one or more reducing sugars, characterized in that the composition comprises or consists of
a) eltrombopag olamine in a therapeutically effective amount and one or more pharmaceutically acceptable excipients in an intragranular composition, and
b) one or more reducing sugars comprising lactose and optionally further one or more pharmaceutically acceptable excipients in an extragranular composition.

A second aspect of the present invention relates to an inventive pharmaceutical tablet composition for use in the treatment or prophylaxis of immune (idiopathic) thrombocytopenic purpura (ITP), thrombocytopenia and/or acquired severe aplastic anaemia (SAA).

A third aspect of the present invention relates to a process of production of an inventive pharmaceutical tablet comprising eltrombopag olamine as active ingredient and one or more pharmaceutically acceptable excipients including one or more reducing sugars, characterized in that the process comprises the following steps:
i. mixing a therapeutically effective amount of eltrombopag olamine and one or more pharmaceutically acceptable excipients,
ii. wet granulation of components mixed in step a) with a suitable granulation fluid, preferably water or an aqueous solution of a binder, in a suitable amount to form an intragranular composition,
iii. mixing one or more reducing sugars comprising lactose and optionally further one or more pharmaceutically acceptable excipients to form an extragranular composition,
iv. mixing the extragranular composition of step c) with the intragranular composition of step b) to form a tablet composition, and
v. compressing the tablet composition of step d) to form the pharmaceutical tablet composition comprising eltrombopag olamine as active ingredient in the intragranular phase and one or more reducing sugars comprising lactose in the extragranular phase.

A fourth aspect of the present invention relates to a pharmaceutical tablet composition comprising eltrombopag olamine as active ingredient and one or more pharmaceutically acceptable excipients including one or more reducing sugars, characterized in that the composition which comprises or consists of
a) eltrombopag olamine in a therapeutically effective amount and one or more pharmaceutically acceptable excipients in an intragranular composition, and
b) one or more reducing sugars comprising lactose and optionally further one or more pharmaceutically acceptable excipients in an extragranular composition,
is obtainable by the inventive production process.

The inventive aspects of the present invention as disclosed hereinbefore can comprise - in case it is reasonable for a person skilled in the art - any possible combination of the preferred inventive embodiments as set out in the dependent claims or disclosed in the following detailed description.

### Detailed description of the invention:

It has surprisingly been found by the present inventors, that the inventive pharmaceutical tablet comprising eltrombopag olamine in the intragranular composition and one or more reducing sugars comprising lactose in the extragranular composition is not subjected to a substantial degradation of the active ingredient eltrombopag olamine in view of Maillard reactions with the reducing sugar and, thus, fulfills the regulatory stability requirements for commercializing the pharmaceutical eltrombopag olamine tablet (see example section Part C, tables 1 and 2). According to a preferred embodiment of the present invention, the inventive pharmaceutical tablet is stable over at least 6 months when stored preferably in Alu-Alu blisters at intermediate or long-term storage conditions, preferably at 25°C / 60% relative humidity (RH) and/or 40°C / 75 % RH. The inventive pharmaceutical tablet composition according to all aspects of the present invention comprises reducing sugars only in the extragranular composition and not in the intragranular composition.

Furthermore, the experimental data provided in table 3 in Part C) of the example section shows that 75 wt.% or more, preferably more than 80 wt.%, more preferably more than 90 wt.% of eltrombopag olamine in the inventive pharmaceutical tablet composition is dissolved within 45 minutes in accordance with the regulatory standard test.

The finding of the present inventors is moreover surprising, as it has been disclosed in the so called Kapsi declaration that eltrombopag olamine compositions comprising lactose are subject to severe degradation and, thus, do not fulfill the stability requirements for commercializing the respective pharmaceutical eltrombopag olamine tablet. The originator accordingly exchanged the lactose excipient by a non-reducing sugar, namely mannitol to fulfill the stability requirements.

Thus, by use of the inventive teaching, namely by separating the eltrombopag olamine active ingredient in the intragranular composition from lactose as the reducing sugar in the extragranular composition, it is possible to maintain the use of lactose as a reducing sugar as diluent constituent in the tablet formulation of eltrombopag olamine.

In the context of the present invention the term "intragranular composition" refers to tablet constituents comprising a therapeutically effective amount of eltrombopag olamine and one or more pharmaceutically acceptable excipients which are wet granulated and thus forming granules to be further processed as intragranular phase of the inventive pharmaceutical tablet composition.

The "extragranular composition" comprising or consisting of one or more reducing sugars comprising lactose and optionally one or more further pharmaceutical excipients forming the extragranular phase in the inventive pharmaceutical tablet composition, is admixed with the granules of the intragranular composition the mixture forms the basis for the tablet composition. This mixture is subsequently compressed to form the inventive pharmaceutical tablet composition.

In the context with all inventive aspects of the present invention, the inventive pharmaceutical tablet composition comprises eltrombopag olamine as the active ingredient in a therapeutically effective amount and optionally one or more further active ingredients. In preferred embodiments of all aspects of the present invention, the active ingredient eltrombopag olamine is used per tablet unit in an amount of 16.05 mg, 32.1 mg, 64.2 mg, 96.3 mg or 128.4 mg eltrombopag olamine (respectively corresponding to 12.5 mg, 25 mg, 50 mg, 75 mg or 100 mg eltrombopag free acid).

In view of eltrombopag olamine as active ingredient, the inventive pharmaceutical tablet composition is effective for use in the treatment or prophylaxis of immune (idiopathic) thrombocytopenic purpura (ITP), thrombocytopenia and/or acquired severe aplastic anaemia (SAA).

Preferably the inventive pharmaceutical tablet composition is indicated for adult chronic immune (idiopathic) thrombocytopenic purpura (ITP) in splenectomised patients who are refractory to other treatments (e.g. corticosteroids, immunoglobulins). The inventive pharmaceutical tablet composition may also be considered as second line treatment for adult non-splenectomised patients where surgery is contraindicated.

The inventive pharmaceutical tablet composition is preferably furthermore indicated in adult patients with chronic hepatitis C virus (HCV) infection for the treatment of thrombocytopenia, where the degree of thrombocytopenia is the main factor preventing the initiation or limiting the ability to maintain optimal interferon-based therapy.

The inventive pharmaceutical tablet composition is preferably furthermore indicated in adult patients with acquired severe aplastic anaemia (SAA) who were either refractory to prior immunosuppressive therapy or heavily pretreated and are unsuitable for haematopoietic stem cell transplantation.

According to all aspects of the present invention suitable reducing sugars, i.e. sugar constituents which generally may form a Maillard reaction with the active ingredient eltrombopag olamine, are used as the one or more reducing sugars acting as diluents in the extragranular composition of the inventive pharmaceutical tablet composition, with the proviso that the one or more reducing sugars comprise lactose. In an alternative or cumulative preferred embodiment of the present invention the one or more reducing sugars are selected from the group consisting of lactose, maltose, glucose, arabinose and fructose, with the proviso that the one or more reducing sugars comprise lactose. More preferably the one or more reducing sugar comprises or consists of lactose selected from the group consisting of lactose monohydrate, anhydrous lactose, spray dried lactose and co-processed lactose. Examples of co-processed lactose are Ludipress® (lactose monohydrate 93 wt.%, 3.5 wt.% povidone (Kollidon®30) and 3.5 wt.% crospovidone (Kollidon® CL); Cellactose® (75% lactose and 25% microcrystalline cellulose MCC); Starlac® (85% lactose and 15% starch) and Combilac® (70% lactose, 20% MCC and 10% corn starch).

The use of lactose as diluent in the extragranular composition is advantageous, as lactose is in particular cheaper than other excipients and thus allows a more economic production of pharmaceutical eltrombopag olamine tablet compositions. Furthermore the use of lactose is advantageous, as a variety of commercially available grades (lactose monohydrate, anhydrous lactose and spray-dried lactose) exists, which enables the skilled person to choose the best fitting grade for the present situation. Accordingly, the skilled person is enabled with respect to all aspects of the present invention to select the best fitting lactose excipient based on the required characteristics for tablet compression. For example, in case the lactose excipient shall also facilitate non-sticking at the compression facilities, lactose comprising a comparatively large particle size will be selected. In case lactose monohydrate is used, fine grades are usually used for the wet granulation process, as they permit better mixing with other excipients.

In accordance with the present invention, the reducing sugar content in the extragranular composition of the inventive pharmaceutical tablet composition is preferably greater than 5 wt.%, more preferably at least 9 wt.%, even more preferably in the range of 15 to 75 wt.%, e.g., 20 to 25 wt.%, 30 to 35 wt.%, 36 to 39 wt.%, 40 to 44 wt.%, 45 to 51 wt.%, 52 to 57 wt.%, 58 to 65 wt.% or alternatively 70 to 75 wt.%, respectively based on the total weight of the pharmaceutical tablet composition. According to an alternative embodiment of the present invention, the inventive pharmaceutical eltrombopag olamine tablets is made using at least 33 to 38 wt.% lactose based on the total weight of the tablet. According to a preferred embodiment the inventive pharmaceutical eltrombopag tablet, the relative content of lactose in comparison to the total weight of the tablet increases with reducing the relative content of the active ingredient eltrombopag olamine. Accordingly, an inventive tablet composition comprising 95.7 mg eltrombopag olamine (equivalent to 75 mg eltrombopag free acid) per tablet unit comprises, e.g. 33 to 38 wt.%, preferably 35 wt.% lactose based on the total weight of the tablet. An inventive tablet composition comprising 63.8 mg eltrombopag olamine (equivalent to 50 mg eltrombopag free acid) per tablet unit comprises, e.g. 45 to 51 wt.%, preferably 47 wt.% lactose based on the total weight of the tablet. An inventive tablet composition comprising 31.9 mg eltrombopag olamine (equivalent to 25 mg eltrombopag free acid) per tablet unit comprises, e.g. 55 to 62 wt.%, preferably 59 wt.% lactose based on the total weight of the tablet. An inventive tablet composition comprising 16 mg eltrombopag olamine (equivalent to 12.5 mg eltrombopag free acid) per tablet unit comprises, e.g. 58 to 65 wt.%, preferably 61 wt.% lactose based on the total weight of the tablet.

According to an alternative or cumulative preferred embodiment of all aspects of the present invention, the intragranular optional extragranular composition of the inventive pharmaceutical tablet composition further comprises or consists of one or more suitable binder agents, preferably one or more binder agents selected from the group consisting of povidone (non-crosslinked polyvinyl pyrrolidone, also called PVP, e.g. commercially available as Plasdone K29/32 or Kollidon® 30), copovidone (vinylpyrrolidone-vinyl acetate copolymer in a ratio of 3:2 by mass, also called PVP/VA, e.g. commercially available as Kollidon® VA64), hydroxypropyl cellulose, low-substituted hydroxypropyl cellulose, hydroxypropyl methylcellulose, hydroxypropyl starch, polyethylene oxide, more preferably one or two binder agents are selected from povidone and copovidone. The use of one or more binder agents is in particular preferred in the intragranular composition. In case co-processed lactose comprising binder agents are used in the extragranular phase, then the amount of binder agent in the intragranular composition may be reduced (see also below paragraph). The additional use of one or more binder agents in the intragranular composition and optionally also in the extragranular composition is preferred, as the respective inventive tablet compositions show a reduced degradation of eltrombopag olamine at higher amounts of 9 wt.% or more, preferably 20 wt.% or more of reducing sugars, in particular lactose as set out in the example section.

According to an alternative of cumulative preferred embodiment of the present invention, the use of co-processed lactose is preferred, as it already comprises a mixture of lactose and one or more further excipients. The one or more further excipients in the co-processed lactose are preferable selected from microcrystalline cellulose (MCC), starch, povidone, and crospovidone (crosslinked polyvinyl pyrrolidone, so called PVPP or polyvinyl polypyrrolidone, e.g. commercially available as Polyplasdone XL or Kollidon® CL). Commercially available co-processed lactose is available as, e.g., Ludipress® which comprises of lactose, povidone (Kollidon® 30) and crospovidone (Kollidon® CL), Cellactose® (lactose with MCC); Starlac® (lactose with starch) and Combilac® (lactose with MCC and starch). In case the further excipients of the co-processed lactose is also present as separate excipient in a different granular phase, then the amount of the separate excipient of the other granular phase may be reduced.

According to an alternative or cumulative preferred embodiment of all aspects of the present invention, the intragranular and/or extragranular composition, preferably the intragranular and extragranular composition further comprises suitable further diluent agents (in addition to the one or more reducing sugars), preferably one or more further diluent agents are selected from the group consisting of erythritol, isomalt, maltitol, xylitol, microcrystalline cellulose, powdered cellulose, pregelatinized starch, starch, lactitol, mannitol, sorbitol, maltodextrin more preferably one, two or more further diluent agents are selected from erythritol, isomalt, maltitol, xylitol, and microcrystalline cellulose, even more preferably the further diluent agents are selected from microcrystalline cellulose and one, two or more selected from erythritol, isomalt, maltitol and xylitol, preferably xylitol.

According to an alternative or cumulative preferred embodiment of all aspects of the present invention, the inventive tablet composition comprises in addition to a reducing sugar in the extragranular composition one or more, preferably one additional diluent agent as set out above. Such a combination is in particular preferred for inventive tablet compositions with a low content of eltrombopag olamine, e.g. 16.05 mg, 32.1 mg, 64.2 mg.

According to an alternative or cumulative preferred embodiment of all aspects of the present invention it may be preferred that the intragranular composition comprises one or more, preferably two further diluent agents as set out above, preferably comprising microcrystalline cellulose and one of the further diluent agents as set out above. Such a combination is preferred as the use of microcrystalline cellulose alone as diluent agent in the intragranular phase may reduce the disintegration time of the inventive tablet composition over storing time.

According to an alternative or cumulative preferred embodiment of all aspects of the present invention it may be preferred that a combination of diluent agents comprises one or more, preferably one soluble and one or more, preferably one insoluble diluent agent in the respective granular phases/compositions. Such a combination further facilitates the disintegration of the inventive tablet, in particular in case no further intragranular disintegrant agent is used in the inventive tablet. Preferred examples of such an inventive combination comprise microcrystalline cellulose insoluble diluent and lactose or xylitol as soluble diluents.

According to an alternative or cumulative preferred embodiment of all aspects of the present invention, the intragranular and/or extragranular composition, preferably the extragranular composition further comprises suitable one or more disintegrant agents, preferably one or more disintegrant agents selected from the group consisting of sodium starch glycolate, crospovidone, and croscarmellose sodium, more preferably one or two selected from sodium starch glycolate, crospovidone and croscarmellose sodium. Generally the disintegrant is applied in the extragranular composition. In case, however, the inventive tablet composition comprises a higher amount of eltrombopag olamine in the intragranular phase and/or an insoluble diluent can be used in the intragranular phase in addition to eltrombopag olamine, then the same or different disintegrant agent may be used also in the intragranular composition, wherein the weight ratios of disintegrant in intra- and extragranular phase is preferably in the range of 1:1 to 1:5, in particular 1:3 or 1:4 or 1:4.6.

As an example, the intragranular phase may comprise no disintegrant agent and the extragranular phase may comprise sodium starch glycolate, crospovidone or croscarmellose sodium or a combination of sodium starch glycolate and crospovidone or sodium starch glycolate and croscarmellose sodium or crospovidone and croscarmellose sodium, more preferably sodium starch glycolate as disintegrant agent(s), wherein the disintegrant agent in total in the extragranular phase is in particular present in a range of 5 to 10 wt.%, preferably 8 wt.% based on the total weight of the inventive tablet composition.

As an alternative example, the intragranular phase may comprise sodium starch glycolate, crospovidone or croscarmellose sodium or a combination of sodium starch glycolate and crospovidone or sodium starch glycolate and croscarmellose sodium or crospovidone and croscarmellose sodium, more preferably sodium starch glycolate as disintegrant agent(s), wherein the disintegrant agent in total in the intragranular phase is in particular present in a weight range of 1 to 5 wt.%, preferably 2 wt.% based on the total weight of the inventive tablet composition, while the extragranular phase may comprise sodium starch glycolate, crospovidone or croscarmellose sodium, or a combination of sodium starch glycolate and crospovidone or sodium starch glycolate and croscarmellose sodium or crospovidone and croscarmellose sodium, wherein the disintegrant agent in total in the extragranular phase is in particular present in a range of 5 to 10 wt.%, preferably 8 wt.% based on the total weight of the inventive tablet composition.

According to an alternative or cumulative preferred embodiment of all aspects of the present invention, the extragranular composition of the inventive pharmaceutical tablet composition further comprises one or more suitable lubricant agents, preferably selected from the group consisting of magnesium stearate, stearic acid, and sodium stearyl fumarate, more preferably magnesium stearate.

According to a further alternative of cumulative preferred embodiment of all aspects of the present invention the pharmaceutical tablet composition is film coated with a coating agent, preferably comprising one or more pharmaceutically acceptable polymers, optionally one or more pharmaceutically acceptable plasticizers, and optionally one or more pharmaceutically acceptable pigments. The one or more polymer agents of the pharmaceutically acceptable coating agent are preferably selected from one or more of the group consisting of hydroxypropyl cellulose, hydroxypropylmethyl cellulose, ethyl cellulose, polyvinyl alcohol, polyethylene glycol, polyethylene glycol-polyvinyl alcohol graftpolymer, and polysorbate. The one or more pigments are preferably selected from the group of titanium dioxide, aluminium lakes, and iron oxides, preferably iron oxide yellow, iron oxide red, and iron oxide black.

According to an alternative or cumulative preferred embodiment of all aspects of the present invention the coating agent comprises or consists of
- hypromellose, macrogol 400, polysorbate 80 and titanium dioxide (commercially available as Opadry® White), preferably for inventive pharmaceutical tablet compositions comprising 16.05 mg or 32.1 mg eltrombopag olamine, or
- hypromellose, macrogol 400, titanium dioxide, iron oxide yellow and iron oxide red (commercially available as Opadry® Brown), preferably for inventive pharmaceutical tablet compositions comprising 64.2 mg eltrombopag olamine, or
- hypromellose, macrogol 400, titanium dioxide, iron oxide red and iron oxide black (commercially available as Opadry® Brown), preferably for inventive pharmaceutical tablet compositions comprising 96.3 mg eltrombopag olamine.

According to alternative or cumulative preferred embodiments of all aspects of the present invention,
i. the total content of binder agent(s) is at least 0.5 wt.% or more, more preferably is in the range of 0.5 to 7 wt.%, and/or
ii. the total content of diluent agent(s) including the reducing sugar amount is at least 5 wt.% or more, preferably is present in the range of 15 to 80 wt.%, preferably 7 to 62 wt.%, and/or
iii. the total content of the disintegrant agent(s) is at least 3 wt.% or more, preferably is in the range of 4 to 15 wt.%, more preferably 5 to 14 wt.%, even more preferably 6 to 12 wt.% and/or
iv. the total content of the lubricant agent(s) is at least 0.5 wt.% or more, preferably is present in the range of 0.5 to 5 wt.%, preferably 0.8 to 3 wt.%, more preferably 0.9 to 1.5 wt.% and/or
v. the total content of the coating agent(s) is at least 2 wt.%, preferably in the range of 3 to 7 wt.%, more preferably 4 to 5 wt.%,
respectively based on the total weight of the pharmaceutical tablet composition.

According to one embodiment of the present invention where the inventive tablet composition comprises 96.3 mg eltrombopag olamine (corresponding to 75 mg eltrombopag free acid) the intragranular composition comprises in addition to eltrombopag olamine at least one binder agent as set out above in an amount of at least 1 wt.% or more, more preferably in the range of 1 wt.% to 5 wt.%, more preferably in the range of 2 wt.% to 4 wt.% based on the total weight of the produced pharmaceutical tablet composition, more preferably the binder agent is povidone. Furthermore, the intragranular composition comprises one, two or more further diluent agents as set out above in a total amount of at least 10 wt.%, more preferably 19 to 22 wt.%, more preferably the further diluent agent is microcrystalline cellulose or a combination of microcrystalline cellulose and xylitol. Furthermore, the intragranular composition comprises one, two or more disintegrant agents as set out above in a total amount of at least 1 wt.%, more preferably 2 wt.%, more preferably the disintegrant agent is sodium starch glycolate or croscarmellose sodium, more preferably sodium starch glycolate. In addition the extragranular composition comprises in addition to the reducing sugar content as diluent, one or more disintegrant agents, preferably in an amount of at least at least 3 wt.% or more, preferably 6 to 12 wt.%, more preferably the disintegrant agent is sodium starch glycolate or croscarmellose sodium or a mixture of sodium starch glycolate and croscarmellose sodium. Optionally the extragranular phase comprises one or more further binder agents, preferably in an amount of at least 0.5 wt.% or more, preferably 2 to 4 wt.%, more preferably povidone and optionally crospovidone. In addition the extragranular composition comprises at least one lubricant, preferably in an amount of at least 0.5 wt.% or more, preferably 0.9 to 1.2 wt.%, more preferably the lubricant is magnesium stearate. Furthermore the inventive tablet composition is film coated comprising suitable coating agents, preferably in an amount of least 2 wt.%, preferably 3 to 5 wt.%. The respective weight amounts as set out before respectively are based on the total weight of the inventive tablet composition. According to a furthermore preferred embodiment, the total weight of the inventive tablet composition is 363 mg.

According to another embodiment of all aspects of the present invention as described herein before, the inventive pharmaceutical tablet composition additionally comprises or consists of a therapeutically effective amount of one or more additional active ingredients. The one or more additional active ingredients may be comprised in the intragranular composition or - in case of prevention of interaction between the active ingredients and eltrombopag olamine - in the extragranular composition. Alternatively, the one or more additional active ingredients are simultaneously or sequentially co-administered in a separate pharmaceutical formulation. In a preferred embodiment, the one or more additional active ingredients are selected from constituents which are effective in the treatment of prophylaxis of thrombocytopenia, preferably adult chronic immune (idiopathic) thrombocytopenic purpura (ITP), acquired severe aplastic anaemia (SAA) and/or chronic hepatitis C virus (HCV) infection.

Accordingly, one or more additional active ingredients are selected from the group of corticosteroids, lithium carbonate, folate, rituximab, and immunoglobuline, in particular anti-Rh (D) immunoglobuline generally used to treat thrombocytopenia, in particular chronic immune (idiopathic) thrombocytopenic purpura (ITP).

Additionally or alternatively one or more additional active ingredients are selected from the group of anti-infective agents, such as antibiotics and antifungal agents, immunosuppressive agents, such as anti-thymocyte globulin (ATG) and cyclosporine-A (CSA) and cytokine agents, such as granulocyte colony-stimulating factor (G-CSF), granulocyte-macrophage colony-stimulating factor (GM-CSF), generally used to treat acquired severe aplastic anaemia (SAA).

Furthermore, one or more additional active ingredients are selected from the group of antiviral agents, such as daclatasvir, elbasvir, grazoprevir, ledipasvir, sofosbuvir, ombitasvir, paritaprevir, ritonavir, dasabuvir, simeprevir, sofosbuvir, velpatasvir, ribavirin, peginterferon alfa-2a or peginterferon alfa-2b generally used to treat chronic hepatitis C virus (HCV) infection.

The properties or the physical and chemical stability of the inventive pharmaceutical tablet composition may be tested in conventional manner, e.g. by measurement of appearance, hardness (or resistance to crushing), disintegration time, dissolution, friability, water content, assay for eltrombopag olamine and/or its degradation products (related substances), and/or uniformity of dosage units or mass after storage at controlled storage conditions; e.g. at intermediate and/or accelerated conditions according to ICH guideline Q1A(R2) (i.e. at 25 °C / 60 % relative humidity (RH) and/or at 40 °C / 75% RH). These tests shall be performed according to applicable pharmaceutical regulatory standards as described e.g. in ICH or EMA guidelines and/or the European Pharmacopeia (EP).

At least some of these attributes, i.e. properties or physical and chemical stability, preferably most of these attributes and most preferably all of these attributes of the inventive pharmaceutical tablet composition are stable over time and different controlled storage conditions. In a preferred embodiment the dissolution (profile) of the inventive pharmaceutical tablet composition according to the present invention, e.g. a tablet or film-coated tablet, is stable over at least 6 months when stored preferably in Alu-Alu blisters at intermediate or long-term storage conditions, i.e. 25 °C / 60 % RH or 40 °C / 75 % RH. More preferably, dissolution and further additional attributes such as, e.g., assay, related substances or uniformity of dosage units or mass are also stable after storage over at least 6 months when stored at intermediate or long-term storage conditions. The term "stable" in the context of the present invention means that a measured value falls within range of specified values determined in accordance with a respective applicable regulatory guideline, e.g. the European Pharmacopeia.

The inventive pharmaceutical tablet composition, preferably film-coated tablet, may vary in shape and be, e.g., round, oval, oblong, cylindrical, caplet shaped or any other suitable shape, preferably it is round or caplet shaped. Furthermore, the inventive pharmaceutical tablet composition comprises means for dividing the tablet, such as scores or engravings.

The inventive pharmaceutical tablet composition, preferably film-coated tablet, has a diameter ranging between 3.5 and 15 mm and most preferably between 5.5 and 7.0 mm for round tablets and the dimension of a caplet is between 9.0 x 3.0 mm and 17.0 x 7.5 mm, preferably it is 12.7 x 5.9 mm. Thickness is ranging from 2.0 to 5.0 mm, preferably between 2.5 and 4.6 mm.

The inventive pharmaceutical tablet composition according to all aspects has a suitable hardness (or resistance to crushing), preferably the film-coated tablets may have a hardness (or resistance to crushing) of at least 40 N, preferably from 50 N to 130 N, more preferably 85 N to 110 N.

Furthermore, the inventive pharmaceutical tablet composition according to all aspects has a suitable disintegration time, preferably the film-coated tablets may have a disintegration time of at least 2 minutes, preferably in the range of 2 to 6 minutes.

Furthermore inventive pharmaceutical tablet composition, preferably film-coated tablet, may be colored and/or marked so as to impart an individual appearance and to make them instantly recognizable. The use of dyes can serve to enhance the appearance as well as to identify the inventive pharmaceutical composition. Dyes suitable for use in pharmacy typically include carotinoids, iron oxides or chlorophyll. The inventive pharmaceutical tablet composition, preferably a film-coated tablet, may be marked using an imprint code.

The inventive pharmaceutical composition may be packed in any conventional packaging known in the art, for example blisters, polypropylene or polyethylene (e.g. HDPE, high density polyethylene) containers or glass bottles. Preferably the inventive tablet composition is preferably packed in a blister known in the art, e.g. sealed aluminum blister (Alu-Alu), Aluminum / Aluminum peel-push blisters or PVC/PE/PVDC/aluminum-blisters.

According to the third aspect of the present invention the inventive process of production of a pharmaceutical tablet comprising eltrombopag olamine as active ingredient and one or more pharmaceutically acceptable excipients including one or more reducing sugars is characterized in that the process comprises or consists of the following steps:
a. mixing a therapeutically effective amount of eltrombopag olamine and one or more pharmaceutically acceptable excipients,
b. wet granulation of components mixed in step a) with a suitable granulation fluid, preferably water or an aqueous solution of a binder, to form an intragranular composition,
c. mixing one or more reducing sugars comprising lactose and optionally further one or more pharmaceutically acceptable excipients to form an extragranular composition,
d. mixing the extragranular composition of step c) with the intragranular composition of step b) to form a tablet composition, and
e. compressing the tablet composition of step d) to form the pharmaceutical tablet composition comprising eltrombopag olamine as active ingredient in the intragranular phase and one or more reducing sugars comprising lactose in the extragranular phase.

The preferred embodiments disclosed with respect to the inventive pharmaceutical tablet composition also apply in combination or separately with respect to the second aspect of the present invention.

Preferably the provided constituents, such as eltrombopag olamine, reducing sugars, disintegrants, binders, diluents and coating agents are sifted prior to use, preferably they are sifted through a # 35 mesh (Tyler Equivalent) screen, which corresponds to a screen with an opening of 0.420 mm in diameter.

According to a preferred embodiment the provided constituents in step a) are loaded into a high shear mixer granulator or an equivalent granulator and preferably mixed for 10, more preferably 5 minutes with impeller at preferably slow speed.

Subsequently, the suitable granulation fluid, preferably water or an aqueous solution of a binder, is added to the mixture of step a) in the high shear mixer granulator and the impeller is preferably used at low speed.

According to a preferred embodiment the granules obtained in step c) after wet granulation are subsequently dried at suitable conditions.

In a further preferred embodiment the granules obtained in step c) preferably dried are subsequently milled, preferably using a Quadro® Comil equipped with a suitable screen.

Subsequently, the (milled) granules forming the intragranular phase and sifted and admixed extragranular constituents, such as the lactose and optionally further constituents of step c) are loaded into a blender and mixed preferably for 10 minutes. In case of using a lubricant, such as magnesium stearate, this constituent is preferably subsequently added to the blender and mixed preferably for further 5 minutes.

Subsequently, the preferably lubricated mixture of step d) is compressed into the inventive pharmaceutical tablet composition preferably on a rotary tablet compression machine using suitable tooling and parameters.

The resulting inventive pharmaceutical tablet composition of step e) is furthermore preferably film coated in a subsequent step f) using one or more pharmaceutically acceptable excipients as suitable coating agent. The degradation of eltrombopag olamine may thereby be further reduced. Preferably the suitable coating agent comprises one or more polymers, optionally one or more plasticizers, and optionally one or more pigments. The preferred coating agents as disclosed with respect to the inventive pharmaceutical tablet composition can also be used with respect to the second aspect of the present invention.

As set out in the Example Section of the present application (see Part C, tables 1 and 2), the amount of impurities measured for the inventive pharmaceutical tablet compositions of the first and third aspect initially after production is below detection limit (BLD). When storing the inventive pharmaceutical tablet compositions under accelerated conditions at open exposure on a petri dish, the amount of impurities is still below detection limit for 40 % relative humidity (RH) and 50°C, 60°C and 70°C. When increasing the relative humidity to 75 % the impurities are detectable at a very low level for 50°C and an increased level for 75°C (see table 1). When using the regulatory accelerated stability assessment standards for Europe, the inventive pharmaceutical tablet compositions are sealed in Alu-Alu blisters and the amount of impurities is still below detection limit both after one or two months. Accordingly, the inventive pharmaceutical tablet compositions of the first and third aspect show a decreased degradation of eltrombopag olamine as active ingredient in comparison to the lactose composition of the prior art (see Kapsi declaration), where the composition already initially after production comprises 1.5 % total impurities. The reduced degradation of eltrombopag olamine may be due to the use of the inventive combination of the lactose with polyvinyl pyrrolidone and/or may be due to the inventive production process according to which the eltrombopag olamine constituent is formulated intragranular with polyvinyl pyrrolidone and the lactose is added together with further polyvinyl pyrrolidone as extragranular excipients.

According to a fourth aspect of the present invention the inventive pharmaceutical tablet composition comprising eltrombopag olamine as active ingredient and one or more pharmaceutically acceptable excipients including one or more reducing sugars, characterized in that the composition comprising or consisting of
a) eltrombopag olamine in a therapeutically effective amount and one or more pharmaceutically acceptable excipients in an intragranular composition, and
b) one or more reducing sugars comprising lactose and optionally further one or more pharmaceutically acceptable excipients in an extragranular composition
is obtainable by use of the inventive production process.

According to a fifth aspect of the present disclosure the use the inventive pharmaceutical tablet composition is disclosed, wherein the pharmaceutical tablet composition is used in the manufacture of a medicament for the treatment or prophylaxis of immune (idiopathic) thrombocytopenic purpura (ITP), thrombocytopenia and/or acquired severe aplastic anaemia (SAA).

According to a sixth aspect of the present disclosure the method of treatment or prophylaxis of immune (idiopathic) thrombocytopenic purpura (ITP), thrombocytopenia and/or acquired severe aplastic anaemia (SAA), comprises administering to a patient in need an inventive pharmaceutical tablet composition is disclosed.

The present invention is explained further with the aid of the following non-limiting examples, illustrating the parameters of and compositions employed within the present invention. Unless stated otherwise, all data, in particular percentages, parts and ratios are by weight.

According to the present invention the individual features of the exemplary embodiments of the inventive pharmaceutical tablet composition and the inventive production process as disclosed in the detailed description or claims of the present application can respectively be separately combined with features of the further exemplary embodiments herein below.

### Examples:

### Part A:

### General Procedure for the wet granulation compression

### Stage A: (Dry Mix)

1. Sifted Eltrombopag olamine and intragranular excipients through #35 mesh.
2. Sifted materials of step-1 loaded into high shear mixer granulator and mixed for 5 minutes with impeller at slow speed.

### Stage B: (Granulation)

3. Step-2 material was granulated with a suitable binder solution, e.g., water or an aqueous solution such as aqueous povidone solution, at impeller slow speed.
4. The granules of step-3 material were dried in a suitable equipment to get required loss on drying.
5. The dried granules of step-4 were milled using Quadro® Comil equipped with suitable screen.

### Stage C: (Blending)

6. Extragranular materials were sifted through #35 mesh.
7. Milled granules of step 5 and sifted extragranular materials of step 6 were loaded into the blender and mixed for 10 min.

### Stage D: (Lubrication)

8. Magnesium stearate sifted through #35 mesh and added to blend mixture of step 7 and lubricated for 5 minutes.
9. The blend mixture of step 8 was compressed into tablets on rotary tablet compression machine using suitable tooling and parameters.

### Stage E: Film-Coating

10. Core tablets of step 9 were coated in coating pan using corresponding Opadry® suspension.

### Part B: Process of production of inventive pharmaceutical tablet compositions

The following inventive pharmaceutical tablet compositions are produced in accordance with the general procedure as set out in Part A above:
**Example F1:** inventive pharmaceutical film-coated tablet composition comprising lactose monohydrate, microcrystalline cellulose, sodium starch glycolate and magnesium stearate in extragranular phase and eltrombopag olamine, microcrystalline cellulose and polyvinyl pyrrolidone (povidone) in intragranular phase

| **Ingredients** | **F1 (mg/unit)** |
|---|---|
| **Stage-A: (Dry Mix)** | |
| Eltrombopag Olamine, *In-house* | 96.902 |
| Cellulose, Microcrystalline *Ph.Eur.* | 82.698 |
| Polyvinyl pyrrolidone, Povidone *Ph.Eur.* (Plasdone K29/32) | 9.600 |

| **Stage-B: (Binder Solution / Granulation)** | |
|---|---|
| Water, purified *Ph.Eur.* | q.s. |

| **Stage-C (Blending)** | |
|---|---|
| Cellulose, Microcrystalline *Ph.Eur.* | 79.300 |
| Microcelac® (Lactose monohydrate in an amount of 75 wt. % and microcrystalline cellulose 25 wt. % respectively based on the total weight of Microcelac®) *In-house* | 48.000 |
| Sodium starch glycolate Type-A *Ph.Eur.* (Explotab) | 28.000 |

| **Stage-D (Blending & Lubrication)** | |
|---|---|
| Magnesium stearate *Ph. Eur.* | 3.500 |
| **Core Tablet weight (mg)** | **348.000** |

| **Stage-E (Film-coating)** | |
|---|---|
| Opadry® Pink *In-house* | 15.000 |
| Water, Purified *Ph.Eur.* | q.s. |
| **Film-coated tablet weight (mg)** | **363.000** |

**Example F2:** inventive pharmaceutical film-coated tablet composition comprising lactose monohydrate, microcrystalline cellulose, sodium starch glycolate and magnesium stearate in extragranular phase and eltrombopag olamine, microcrystalline cellulose and polyvinyl pyrrolidone (povidone) in intragranular phase

| **Ingredients** | **F2 (mg/unit)** |
|---|---|
| **Stage-A: (Dry Mix)** | |
| Eltrombopag Olamine, *In-house* | 96.902 |
| Cellulose, Microcrystalline *Ph.Eur.* | 118.698 |
| Polyvinyl pyrrolidone, Povidone *Ph.Eur.* (Plasdone K29/32) | 9.600 |

| **Stage-B: (Binder Solution / Granulation)** | |
|---|---|
| Water, purified *Ph.Eur.* | q.s. |

| Stage-C **(Blending)** | |
|---|---|
| Cellulose, Microcrystalline *Ph.Eur.* | 79.300 |
| Lactose monohydrate *Ph.Eur.* (Tablettose 100) | 12.000 |
| Sodium starch glycolate Type-A *Ph.Eur.* (Explotab) | 28.000 |

| **Stage-D (Blending & Lubrication)** | |
|---|---|
| Magnesium stearate *Ph. Eur.* | 3.500 |
| **Core Tablet weight (mg)** | **348.000** |

| **Stage-E (Film-coating)** | |
|---|---|
| Opadry® Pink *In-house* | 15.000 |
| Water, Purified *Ph.Eur.* | q.s. |
| **Film-coated tablet weight (mg)** | **363.000** |

**Example F3:** inventive pharmaceutical film-coated tablet composition comprising lactose monohydrate, microcrystalline cellulose, polyvinyl pyrrolidone (povidone), crosslinked insoluble polyvinylpyrrolidone (crospovidone), sodium starch glycolate and magnesium stearate in extragranular phase and eltrombopag olamine, microcrystalline cellulose and polyvinyl pyrrolidone (povidone) in intragranular phase

| **Ingredients** | **F3 (mg/unit)** |
|---|---|
| **Stage-A: (Dry Mix)** | |
| Eltrombopag Olamine, *In-house* | 96.902 |
| Cellulose, Microcrystalline *Ph.Eur.* | 91.698 |
| Polyvinyl pyrrolidone, Povidone *Ph.Eur.* (Plasdone K29/32) | 9.600 |

| **Stage-B: (Binder Solution / Granulation)** | |
|---|---|
| Water, purified *Ph.Eur.* | q.s. |

| **Stage-C (Blending)** | |
|---|---|
| Cellulose, Microcrystalline *Ph.Eur.* | 79.300 |
| Ludipress® (Lactose monohydrate in an amount of 93 wt. %, polyvinyl pyrrolidone, Povidone, Kollidon® 30 in an amount of 3.5 wt. % and crosslinked insoluble polyvinylpyrrolidone, Crospovidone, Kollidon® CL 3.5 wt. % respectively based on the total weight of Ludipress®) | 39.000 |
| Sodium starch glycolate Type-A *Ph.Eur.* (Explotab) | 28.000 |

| **Stage-D (Blending & Lubrication)** | |
|---|---|
| Magnesium stearate *Ph.Eur.* | 3.500 |
| **Core Tablet weight (mg)** | **348.000** |

| **Stage-E (Film-coating)** | |
|---|---|
| Opadry® Pink *In-house* | 15.000 |
| Water, Purified *Ph.Eur.* | q.s. |
| **Film-coated tablet weight (mg)** | **363.000** |

**Example F4:** inventive pharmaceutical film-coated tablet composition comprising lactose monohydrate, microcrystalline cellulose, sodium starch glycolate and magnesium stearate in extragranular phase and eltrombopag olamine, microcrystalline cellulose and polyvinyl pyrrolidone (povidone) in intragranular phase

| **Ingredients** | **F4 (mg/unit)** |
|---|---|
| **Stage-A: (Dry Mix)** | |
| Eltrombopag Olamine, *In-house* | 96.902 |
| Cellulose, Microcrystalline *Ph.Eur.* | 94.698 |
| Polyvinyl pyrrolidone, Povidone *Ph.Eur.* (Plasdone K29/32) | 9.600 |

| **Stage-B: (Binder Solution / Granulation)** | |
|---|---|
| Water, purified *Ph.Eur.* | q.s. |

| **Stage-C (Blending)** | |
|---|---|
| Cellulose, Microcrystalline *Ph.Eur.* | 79.300 |
| Lactose monohydrate *Ph.Eur.* (Tablettose 100) | 36.000 |
| Sodium starch glycolate Type-A *Ph.Eur.* (Explotab) | 28.000 |

| **Stage-D (Blending & Lubrication)** | |
|---|---|
| Magnesium stearate *Ph.Eur.* | 3.500 |
| **Core Tablet weight (mg)** | **348.000** |

| **Stage-E (Film-coating)** | |
|---|---|
| Opadry® Pink *In-house* | 15.000 |
| Water, Purified *Ph.Eur.* | q.s. |
| **Film-coated tablet weight (mg)** | **363.000** |

**Example F5:** inventive pharmaceutical film-coated tablet composition comprising lactose monohydrate, xylitol, sodium starch glycolate and magnesium stearate in extragranular phase and eltrombopag olamine, microcrystalline cellulose and polyvinyl pyrrolidone (povidone) in intragranular phase

| **Ingredients** | **F5 (mg/unit)** |
|---|---|
| **Stage-A: (Dry Mix)** | |
| Eltrombopag Olamine, *In-house* | 96.902 |
| Cellulose, Microcrystalline *Ph.Eur.* | 73.000 |
| Polyvinyl pyrrolidone, Povidone *Ph.Eur.* (Plasdone K29/32) | 9.600 |

| **Stage-B: (Binder Solution / Granulation)** | |
|---|---|
| Water, purified *Ph.Eur.* | q.s. |

| **Stage-C (Blending)** | |
|---|---|
| Xylitol *Ph.Eur.* | 92.998 |
| Lactose monohydrate *Ph.Eur.* (Tablettose 100) | 44.000 |
| Sodium starch glycolate Type-A *Ph.Eur.* (Explotab) | 28.000 |

| **Stage-D (Blending & Lubrication)** | |
|---|---|
| Magnesium stearate *Ph.Eur.* | 3.500 |
| **Core Tablet weight (mg)** | **348.000** |

| **Stage-E (Film-coating)** | |
|---|---|
| Opadry® Brown *In-house* | 15.000 |
| Water, Purified *Ph.Eur.* | q.s. |
| **Film-coated tablet weight (mg)** | **363.000** |

**Example F6:** inventive pharmaceutical film-coated tablet composition comprising lactose monohydrate, xylitol, sodium starch glycolate and magnesium stearate in extragranular phase and eltrombopag olamine, microcrystalline cellulose and polyvinyl pyrrolidone (povidone) in intragranular phase

| **Ingredients** | **F6 (mg/unit)** |
|---|---|
| **Stage-A: (Dry Mix)** | |
| Eltrombopag Olamine, *In-house* | 96.902 |
| Cellulose, Microcrystalline *Ph.Eur.* | 73.000 |
| Polyvinyl pyrrolidone, Povidone *Ph.Eur.* (Plasdone K29/32) | 9.600 |

| **Stage-B: (Binder Solution / Granulation)** | |
|---|---|
| Water, purified *Ph.Eur.* | q.s. |

| **Stage-C (Blending)** | |
|---|---|
| Xylitol *Ph.Eur.* | 81.998 |
| Lactose monohydrate *Ph.Eur.* (Tablettose 100) | 55.000 |
| Sodium starch glycolate Type-A *Ph.Eur.* (Explotab) | 28.000 |

| **Stage-D (Blending & Lubrication)** | |
|---|---|
| Magnesium stearate *Ph.Eur.* | 3.500 |
| **Core Tablet weight (mg)** | **348.000** |

| **Stage-E (Film-coating)** | |
|---|---|
| Opadry® Brown *In-house* | 15.000 |
| Water, Purified *Ph.Eur.* | q.s. |
| **Film-coated tablet weight (mg)** | **363.000** |

**Example F7:** inventive pharmaceutical film-coated tablet composition comprising lactose monohydrate, xylitol, sodium starch glycolate and magnesium stearate in extragranular phase and eltrombopag olamine, microcrystalline cellulose and polyvinyl pyrrolidone (povidone) in intragranular phase

| **Ingredients** | **F7 (mg/unit)** |
|---|---|
| **Stage-A: (Dry Mix)** | |
| Eltrombopag Olamine, *In-house* | 96.902 |
| Cellulose, Microcrystalline *Ph.Eur.* | 73.000 |
| Polyvinyl pyrrolidone, Povidone *Ph.Eur.* (Plasdone K29/32) | 9.600 |

| **Stage-B: (Binder Solution / Granulation)** | |
|---|---|
| Water, purified *Ph. Eur.* | q.s. |

| **Stage-C (Blending)** | |
|---|---|
| Xylitol *Ph. Eur.* | 9.998 |
| Lactose monohydrate *Ph.Eur.* (Tablettose 100) | 127.000 |
| Sodium starch glycolate Type-A *Ph.Eur.* (Explotab) | 28.000 |

| **Stage-D (Blending & Lubrication)** | |
|---|---|
| Magnesium stearate *Ph.Eur.* | 3.500 |
| **Core Tablet weight (mg)** | **348.000** |

| **Stage-E (Film-coating)** | |
|---|---|
| Opadry® Brown *In-house* | 15.000 |
| Water, Purified *Ph.Eur.* | q.s. |
| **Film-coated tablet weight (mg)** | **363.000** |

**Examples F8 and F9:** inventive pharmaceutical film-coated tablet compositions comprising co-processed lactose comprising lactose monohydrate, polyvinyl pyrrolidone (povidone) and crosslinked insoluble polyvinyl pyrrolidone (crospovidone), sodium starch glycolate and magnesium stearate in extragranular phase and eltrombopag olamine, microcrystalline cellulose and polyvinyl pyrrolidone (povidone) in intragranular phase

| **Ingredients** | **F8 (mg/unit)** | **F9 (mg/unit)** |
|---|---|---|
| **Stage-A: (Dry Mix)** | | |
| Eltrombopag Olamine, *In-house* | 96.902 | 95.703 |
| Cellulose, Microcrystalline *Ph.Eur.* | 73.000 | 72.365 |
| Polyvinyl pyrrolidone, Povidone *Ph.Eur.* (Plasdone K29/32) | 9.600 | 9.600 |

| **Stage-B: (Binder Solution / Granulation)** | | |
|---|---|---|
| Water, purified *Ph.Eur.* | q.s. | q.s. |

| **Stage-C (Blending)** | | |
|---|---|---|
| Ludipress® (Lactose monohydrate in an amount of 93 wt. %, polyvinyl pyrrolidone, Povidone, Kollidon® 30 in an amount of 3.5 wt. % and crosslinked insoluble polyvinylpyrrolidone, Crospovidone, Kollidon® CL 3.5 wt. % respectively based on the total weight of Ludipress®) | 137.000 | 138.832 |
| Sodium starch glycolate Type-A *Ph.Eur.* (Explotab) | 28.000 | 28.000 |

| **Stage-D (Blending & Lubrication)** | | |
|---|---|---|
| Magnesium stearate *Ph.Eur.* | 3.500 | 3.500 |
| **Core Tablet weight (mg)** | **348.000** | **348.000** |

| **Stage-E (Film-coating)** | | |
|---|---|---|
| Opadry® Brown *In-house* | 15.000 | 15.000 |
| Water, Purified *Ph.Eur.* | q.s. | q.s. |
| **Film-coated tablet weight (mg)** | **363.000** | **363.000** |

**Examples F10 and F11:** inventive pharmaceutical film-coated tablet compositions comprising lactose monohydrate, polyvinyl pyrrolidone (povidone), sodium starch glycolate and magnesium stearate in extragranular phase and eltrombopag olamine, microcrystalline cellulose and polyvinyl pyrrolidone (povidone) in intragranular phase

| **Ingredients** | **F10 (mg/unit)** | **F11 (mg/unit)** |
|---|---|---|
| **Stage-A: (Dry Mix)** | | |
| Eltrombopag Olamine, *In-house* | 96.902 | 95.703 |
| Cellulose, Microcrystalline *Ph.Eur.* | 73.000 | 72.365 |
| Polyvinyl pyrrolidone, Povidone *Ph.Eur.* (Plasdone K29/32) | 9.600 | 9.600 |

| **Stage-B: (Binder Solution / Granulation)** | | |
|---|---|---|
| Water, purified *Ph.Eur.* | q.s. | q.s. |

| **Stage-C (Blending)** | | |
|---|---|---|
| Lactose monohydrate *Ph.Eur.* (Tablettose 100) | 127.000 | 128.832 |
| Polyvinyl pyrrolidone, Povidone *Ph.Eur.* (Plasdone K29/32) | 9.998 | 10.000 |
| Sodium starch glycolate Type-A *Ph.Eur.* (Explotab) | 28.000 | 28.000 |

| **Stage-D (Blending & Lubrication)** | | |
|---|---|---|
| Magnesium stearate *Ph.Eur.* | 3.500 | 3.500 |
| **Core Tablet weight (mg)** | **348.000** | **348.000** |

| **Stage-E (Film-coating)** | | |
|---|---|---|
| Opadry® Brown *In-house* | 15.000 | 15.000 |
| Water, Purified *Ph.Eur.* | q.s. | q.s. |
| **Film-coated tablet weight (mg)** | **363.000** | **363.000** |

**Example F12:** inventive pharmaceutical film-coated tablet composition comprising lactose monohydrate, microcrystalline cellulose, sodium starch glycolate and magnesium stearate in extragranular phase and eltrombopag olamine, microcrystalline cellulose, xylitol and polyvinyl pyrrolidone (povidone) in intragranular phase

| **Ingredients** | **F12 (mg/unit)** |
|---|---|
| **Stage-A: (Dry Mix)** | |
| Eltrombopag Olamine, *In-house* | 96.251 |
| Cellulose, Microcrystalline *Ph.Eur.* (Comprecel M 102 D+) | 45.000 |
| Xylitol | 80.000 |
| Polyvinyl pyrrolidone, Povidone *Ph.Eur.* (Plasdone K29/32) | 10.000 |

| **Stage-B: (Binder Solution / Granulation)** | |
|---|---|
| Water, purified *Ph.Eur.* | q.s. |

| **Stage-C (Blending)** | |
|---|---|
| Cellulose, Microcrystalline *Ph.Eur.* (Comprecel M 102 D+) | 27.900 |
| Lactose monohydrate *Ph.Eur.* | 57.349 |
| Sodium starch glycolate Type-A *Ph.Eur.* (Explotab) | 28.000 |

| **Stage-D (Blending & Lubrication)** | |
|---|---|
| Magnesium stearate *Ph.Eur.* | 3.500 |
| **Core Tablet weight (mg)** | **348.000** |

| **Stage-E (Film-coating)** | |
|---|---|
| Opadry® Brown *In-house* | 15.000 |
| Water, Purified *Ph.Eur.* | q.s. |
| **Film-coated tablet weight (mg)** | **363.000** |

**Example F13:** inventive pharmaceutical film-coated tablet composition comprising anhydrous lactose, sodium starch glycolate and magnesium stearate in extragranular phase and eltrombopag olamine, microcrystalline cellulose, sodium starch glycolate and polyvinyl pyrrolidone (povidone) in intragranular phase

| **Ingredients** | **F13 (mg/unit)** |
|---|---|
| **Stage-A: (Dry Mix)** | |
| Eltrombopag Olamine, *In-house* | 96.338 |
| Cellulose, Microcrystalline *Ph. Eur.* | 73.000 |
| Polyvinyl pyrrolidone, Povidone *Ph.Eur.* (Plasdone K29/32) | 20.000 |
| Sodium starch glycolate Type-A *Ph.Eur.* (Explotab) | 14.000 |

| **Stage-B: (Binder Solution / Granulation)** | |
|---|---|
| Water, purified *Ph. Eur.* | q.s. |

| **Stage-C (Blending)** | |
|---|---|
| Lactose anhydrous *Ph.Eur.* (Supertab 21 AN) | 127.162 |
| Sodium starch glycolate Type-A *Ph.Eur.* (Explotab) | 14.000 |

| **Stage-D (Blending & Lubrication)** | |
|---|---|
| Magnesium stearate *Ph.Eur.* | 3.500 |
| **Core Tablet weight (mg)** | **348.000** |

| **Stage-E (Film-coating)** | |
|---|---|
| Opadry® Brown *In-house* | 15.000 |
| Water, Purified *Ph.Eur.* | q.s. |
| **Film-coated tablet weight (mg)** | **363.000** |

**Example F14:** inventive pharmaceutical film-coated tablet composition comprising lactose monohydrate, microcrystalline cellulose, sodium starch glycolate and magnesium stearate in extragranular phase and eltrombopag olamine, microcrystalline cellulose, and polyvinyl pyrrolidone (povidone) in intragranular phase

| | |
|---|---|
| **Ingredients** | **F14 (mg/unit)** |
| **Stage-A: (Dry Mix)** | |
| Eltrombopag Olamine, *In-house* | 96.251 |
| Cellulose, Microcrystalline *Ph.Eur.* (Comprecel M 102 D+) | 18.269 |
| Sodium starch glycolate Type-A *Ph.Eur.* (Explotab) | 7.000 |

| **Stage-B: (Binder Solution / Granulation)** | |
|---|---|
| Polyvinyl pyrrolidone, Povidone *Ph.Eur.* (Plasdone K29/32) | 10.000 |
| Water, purified *Ph.Eur.* | q.s. |

| **Stage-C (Blending)** | |
|---|---|
| Cellulose, Microcrystalline *Ph.Eur.* (Comprecel M 102 D+) | 54.000 |
| Lactose monohydrate *Ph.Eur.* (Flowlac 90) | 137.980 |
| Sodium starch glycolate Type-A *Ph.Eur.* (Explotab) | 21.000 |

| **Stage-D (Blending & Lubrication)** | |
|---|---|
| Magnesium stearate *Ph.Eur.* | 3.500 |
| **Core Tablet weight (mg)** | **348.000** |

| **Stage-E (Film-coating)** | |
|---|---|
| Opadry® Brown *In-house* | 15.000 |
| Water, Purified *Ph.Eur.* | q.s. |
| **Film-coated tablet weight (mg)** | **363.000** |

**Example F15:** inventive pharmaceutical film-coated tablet composition comprising lactose monohydrate, microcrystalline cellulose, croscarmellose sodium and magnesium stearate in extragranular phase and eltrombopag olamine, microcrystalline cellulose and polyvinyl pyrrolidone (povidone) in intragranular phase

| **Ingredients** | **F15 (mg/unit)** |
|---|---|
| **Stage-A: (Dry Mix)** | |
| Eltrombopag Olamine, *In-house* | 96.251 |
| Cellulose, Microcrystalline *Ph.Eur.* (Comprecel M 102 D+) | 36.269 |

| **Stage-B: (Binder Solution / Granulation)** | |
|---|---|
| Polyvinyl pyrrolidone, Povidone *Ph.Eur.* (Plasdone K29/32) | 10.000 |
| Water, purified *Ph. Eur.* | q.s. |

| **Stage-C (Blending)** | |
|---|---|
| Cellulose, Microcrystalline *Ph.Eur.* (Comprecel M 102 D+) | 36.000 |
| Lactose monohydrate *Ph.Eur.* (Flowlac 90) | 137.980 |
| Croscarmellose sodium *Ph.Eur.* (Ac-Di-Sol) | 28.000 |

| **Stage-D (Blending & Lubrication)** | |
|---|---|
| Magnesium stearate *Ph.Eur.* | 3.500 |
| **Core Tablet weight (mg)** | **348.000** |

| **Stage-E (Film-coating)** | |
|---|---|
| Opadry® Brown *In-house* | 15.000 |
| Water, Purified *Ph.Eur.* | q.s. |
| **Film-coated tablet weight (mg)** | **363.000** |

**Example F16:** inventive pharmaceutical film-coated tablet composition comprising lactose monohydrate, microcrystalline cellulose, crospovidone and magnesium stearate in extragranular phase and eltrombopag olamine, microcrystalline cellulose and polyvinyl pyrrolidone (povidone) in intragranular phase

| **Ingredients** | **F16 (mg/unit)** |
|---|---|
| **Stage-A: (Dry Mix)** | |
| Eltrombopag Olamine, *In-house* | 96.251 |
| Cellulose, Microcrystalline *Ph.Eur.* (Comprecel M 102 D+) | 36.269 |

| **Stage-B: (Binder Solution / Granulation)** | |
|---|---|
| Polyvinyl pyrrolidone, Povidone *Ph.Eur.* (Plasdone K29/32) | 10.000 |
| Water, purified *Ph.Eur.* | q.s. |

| **Stage-C (Blending)** | |
|---|---|
| Cellulose, Microcrystalline *Ph.Eur.* (Comprecel M 102 D+) | 36.000 |
| Lactose monohydrate *Ph.Eur.* (Flowlac 90) | 137.980 |
| Crospovidone *Ph.Eur.* | 28.000 |

| **Stage-D (Blending & Lubrication)** | |
|---|---|
| Magnesium stearate *Ph.Eur.* | 3.500 |
| **Core Tablet weight (mg)** | **348.000** |

| **Stage-E (Film-coating)** | |
|---|---|
| Opadry® Brown *In-house* | 15.000 |
| Water, Purified *Ph.Eur.* | q.s. |
| **Film-coated tablet weight (mg)** | **363.000** |

**Examples F17 to F20:** inventive pharmaceutical film-coated tablet composition comprising lactose monohydrate, polyvinyl pyrrolidone (povidone), sodium starch glycolate and magnesium stearate in extragranular phase and 16 mg, 31.1 mg or 63.2 mg eltrombopag olamine (equivalent to 12.5 mg, 25 mg or 50 mg eltrombopag) in combination with microcrystalline cellulose and povidone in intragranular phase

| **Fngredients** | **F17** | **F18** | **F19** | **F20** |
|---|---|---|---|---|
| **Stage-A: (Dry Mix)** | **(mg/unit)** | **(mg/unit)** | **(mg/unit)** | **(mg/unit)** |
| Eltrombopag Olamine, *In-house* | 63.167 | 31.084 | 16.042 | 16.042 |
| Cellulose, Microcrystalline *Ph.Eur. (Comprecel M 102 D*+*)* | 73.000 | 73.00 | 73.00 | 36.500 |
| Povidone *Ph.Eur.* (Plasdone K29/32) | 9.600 | 9.60 | 9.60 | 4.800 |
| Water, Purified *Ph.Eur.* | q.s. | q.s. | q.s. | q.s. |

| **Stage-B (Blending)** | | | | |
|---|---|---|---|---|
| Lactose monohydrate *Ph.Eur.* (Tablettose 100) | 160.733 | 192.816 | 207.858 | 95.908 |
| Povidone *Ph.Eur.* (Plasdone K29/32) | 10.000 | 10.000 | 10.000 | 5.000 |
| Sodium starch glycolate Type-A *Ph.Eur.* (Explotab) | 28.000 | 28.000 | 28.000 | 14.000 |

| **Stage-C (Lubrication)** | | | | |
|---|---|---|---|---|
| Magnesium stearate *Ph.Eur.* | 3.500 | 3.500 | 3.500 | 1.750 |
| **Core Tablet weight (mg)** | **348.000** | **348.000** | **348.000** | **348.000** |

| **Stage-D (Film-coating)** | | | | |
|---|---|---|---|---|
| Opadry® Brown *In-house* | 15.000 | - | - | - |
| Opadry® White *In-house* | - | 15.000 | 15.000 | 7.500 |
| Water, Purified *Ph.Eur.* | q.s. | q.s. | q.s. | q.s. |
| **Film-coated tablet weight (mg)** | **363.000** | **363.000** | **363.000** | **363.000** |

**Examples F21 and F22:** inventive pharmaceutical film-coated tablet composition comprising Ludipress® (93 wt% lactose monohydrate, 3.5 wt% polyvinyl pyrrolidone (Kollidon 30) and 3.5 wt% crospovidone (Kollidon CL)) and sodium starch glycolate and magnesium stearate in extragranular phase and 16 mg eltrombopag olamine (equivalent to 12.5 mg eltrombopag) in combination with microcrystalline cellulose and povidone in intragranular phase

| **Ingredients** | **F21** | **F22** |
|---|---|---|
| **Stage-A: (Dry Mix)** | **(mg/unit)** | **(mg/unit)** |
| Eltrombopag Olamine, *In-house* | 16.042 | 16.042 |
| Cellulose, Microcrystalline *Ph.Eur.* (Comprecel M 102 D+) | 73.000 | 36.500 |
| Povidone *Ph.Eur.* (Plasdone K29/32) | 9.600 | 4.80 |
| Water, Purified *Ph.Eur.* | q.s. | q.s. |

| **Stage-B (Blending)** | | |
|---|---|---|
| Ludipress® (93% Lactose monohydrate, 3.5% Povidone, and 3.5% Crospovidone) | 217.858 | 100.908 |
| Povidone *Ph.Eur.* (Plasdone K29/32) | - | - |
| Sodium starch glycolate Type-A *Ph.Eur.* (Explotab) | 28.000 | 14.000 |

| Stage-C (Lubrication) | | |
|---|---|---|
| Magnesium stearate *Ph.Eur.* | 3.500 | 1.750 |
| **Core Tablet weight (mg)** | **348.000** | **174.000** |

| **Stage-D (Film-coating)** | | |
|---|---|---|
| Opadry ® White *In-house* | 15.000 | 7.500 |
| Water, Purified *Ph.Eur.* | q.s. | q.s. |
| **Film-coated tablet weight (mg)** | **363.000** | **181.500** |

**Examples F23 and F24:** inventive pharmaceutical film-coated tablet composition comprising lactose monohydrate, copovidone, sodium starch glycolate and magnesium stearate in extragranular phase and 16 mg eltrombopag olamine (equivalent to 12.5 mg eltrombopag) in combination with microcrystalline cellulose and copovidone in intragranular phase

| **Ingredients** | **F23** | **F24** |
|---|---|---|
| **Stage-A: (Dry Mix)** | **(mg/unit)** | **(mg/unit)** |
| Eltrombopag Olamine, *In-house* | 16.042 | 16.042 |
| Cellulose, Microcrystalline *Ph.Eur. (Comprecel M 102 D*+*)* | 73.000 | 36.500 |
| Copovidone *Ph.Eur.* (Plasdone S 630) | 9.600 | 4.800 |
| Water, Purified *Ph.Eur.* | q.s. | q.s. |

| **Stage-B (Blending)** | | |
|---|---|---|
| Lactose monohydrate *Ph.Eur.* (Tablettose 100) | 207.858 | 95.908 |
| Copovidone *Ph.Eur.* (Plasdone S 630) | 10.000 | 5.000 |
| Sodium starch glycolate Type-A *Ph.Eur.* (Explotab) | 28.000 | 14.000 |

| **Stage-C (Lubrication)** | | |
|---|---|---|
| Magnesium stearate *Ph.Eur.* | 3.500 | 1.750 |
| **Core Tablet weight (mg)** | **348.000** | **174.000** |

| **Stage-D (Film-coating)** | | |
|---|---|---|
| Opadry ® White *In-house* | 15.000 | 7.500 |
| Water, Purified *Ph.Eur.* | q.s. | q.s. |
| **Film-coated tablet weight (mg)** | **363.000** | **181.500** |

**Examples F25 to F28:** inventive pharmaceutical film-coated tablet composition comprising lactose monohydrate, microcrystalline cellulose, croscarmellose sodium, sodium starch glycolate and magnesium stearate in extragranular phase and 16 mg, 31.9 mg, 63.2 mg or 95.7 mg eltrombopag olamine (equivalent to 12.5 mg, 25 mg, 50 mg or 75 mg eltrombopag) in combination with microcrystalline cellulose, polyvinyl pyrrolidone and sodium starch glycolate in intragranular phase

| **Ingredients** | **F25** | **F26** | **F27** | **F28** |
|---|---|---|---|---|
| **Stage-A: (Dry Mix)** | **(mg/unit)** | **(mg/unit)** | **(mg/unit)** | **(mg/unit)** |
| Eltrombopag Olamine, *In-house* | 95.703 | 63.802 | 31.901 | 15.951 |
| Cellulose, Microcrystalline *Ph.Eur. (Comprecel M 101 D*+*)* | 18.000 | 12.000 | 6.000 | 3.000 |
| Sodium starch glycolate Type-A *Ph.Eur.* (Explotab) | 7.000 | 4.670 | 2.333 | 1.167 |

| **Stage-B (Binder solution)** | | | | |
|---|---|---|---|---|
| Povidone *Ph.Eur.* (Plasdone K29/32) | 10.000 | 6.667 | 3.333 | 1.167 |
| Water, Purified *Ph.Eur.* | q.s. | q.s. | q.s. | q.s. |

| **Stage-B (Blending)** | | | | |
|---|---|---|---|---|
| Cellulose, Microcrystalline *Ph.Eur.* (Avicel PH 200) | 54.000 | 36.000 | 18.000 | 9.000 |
| Lactose monohydrate *Ph.Eur.* (Tablettose 80) | 126.797 | 84.530 | 42.267 | 21.133 |
| Croscarmellose sodium *Ph.Eur. (Ac-di-sol SD 711)* | 5.000 | 3.333 | 1.667 | 0.833 |
| Sodium starch glycolate Type-A *Ph.Eur.* (Explotab) | 28.000 | 18.667 | 9.333 | 4.667 |

| **Stage-C (Lubrication)** | | | | |
|---|---|---|---|---|
| Magnesium stearate *Ph.Eur.* | 3.5000 | 2.333 | 1.167 | 0.583 |
| **Core Tablet weight (mg)** | **348.000** | **232.000** | **116.000** | **58.000** |

| **Continuation of Ingredients** | **F25** | **F26** | **F27** | **F28** |
|---|---|---|---|---|
| **Stage-D (Film-coating)** | | | | |
| Opadry ® Brown 85F565229 *In-house* | 15.000 | - | - | - |
| Opadry ® Brown 85F565230 *In-house* | - | 10.000 | - | - |
| Opadry ® White *In-house* | - | - | 5.000 | 2.500 |
| Water, Purified *Ph.Eur.* | q.s. | q.s. | q.s. | q.s. |
| **Film-coated tablet weight (mg)** | **363.000** | **242.000** | **121.000** | **60.500** |

**Examples F29 to F32:** inventive pharmaceutical film-coated tablet composition comprising lactose monohydrate, microcrystalline cellulose, croscarmellose sodium, sodium starch glycolate and magnesium stearate in extragranular phase and 16 mg, 31.9 mg, or 63.2 mg eltrombopag olamine (equivalent to 12.5 mg, 25 mg, or 50 mg eltrombopag) in combination with microcrystalline cellulose, polyvinyl pyrrolidone (povidone) and sodium starch glycolate in intragranular phase

| **Ingredients** | **F29** | **F30** | **F31** |
|---|---|---|---|
| **Stage-A: (Dry Mix)** | **(mg/unit)** | **(mg/unit)** | **(mg/unit)** |
| Eltrombopag Olamine, *In-house* | 63.802 | 31.901 | 15.951 |
| Cellulose, Microcrystalline *Ph.Eur. (Comprecel M 101 D+)* | 12.000 | 6.000 | 3.000 |
| Sodium starch glycolate Type-A *Ph.Eur.* (Explotab) | 4.667 | 2.333 | 1.167 |

| Stage-B (Binder solution) | | | |
|---|---|---|---|
| Povidone *Ph.Eur.* (Plasdone K29/32) | 6.667 | 3.333 | 1.667 |
| Water, Purified *Ph.Eur.* | q.s. | q.s. | q.s. |

| **Stage-B (Blending)** | | | |
|---|---|---|---|
| Cellulose, Microcrystalline *Ph.Eur.* (Avicel PH 200) | 54.000 | 54.000 | 27.000 |
| Lactose monohydrate *Ph.Eur.* (Tablettose 80) | 170.365 | 213.932 | 106.966 |
| Croscarmellose sodium *Ph.Eur. (Ac-di-sol SD 711)* | 5.000 | 5.000 | 2.500 |
| Sodium starch glycolate Type-A *Ph.Eur.* (Explotab) | 28.000 | 28.000 | 14.000 |

| **Stage-C (Lubrication)** | | | |
|---|---|---|---|
| Magnesium stearate *Ph.Eur.* | 3.500 | 3.500 | 1.750 |
| **Core tablet weight (mg)** | **348.000** | **348.000** | **174.000** |

| **Stage-D (Film-coating)** | | | |
|---|---|---|---|
| Opadry ® Brown *In-house* | 15.000 | - | - |
| Opadry ®White *In-house* | - | 15.000 | 7.500 |
| Water, Purified *Ph.Eur.* | q.s. | q.s. | q.s. |
| **Film-coated tablet weight (mg)** | **363.000** | **363.000** | **181.500** |

**Example F32:** inventive pharmaceutical film-coated tablet composition comprising lactose monohydrate, mannitol, sodium starch glycolate and magnesium stearate in extragranular phase and 96.25 mg eltrombopag olamine (equivalent to 75 mg eltrombopag), microcrystalline cellulose, sodium starch glycolate, and polyvinyl pyrrolidone (povidone) in intragranular phase

| **Ingredients** | **F32** |
|---|---|
| **Stage-A: (Dry Mix)** | **(mg/unit)** |
| Eltrombopag Olamine, *In-house* | 96.251 |
| Cellulose, Microcrystalline *Ph.Eur.* (Comprecel M 102 D+) | 18.269 |
| Sodium starch glycolate Type-A *Ph.Eur.* (Explotab) | 7.000 |

| **Stage-B: (Binder Solution / Granulation)** | |
|---|---|
| Polyvinyl pyrrolidone, Povidone *Ph.Eur.* (Plasdone K29/32) | 10.000 |
| Water, purified *Ph.Eur.* | q.s. |

| **Stage-C (Blending)** | |
|---|---|
| Mannitol *Ph.Eur.* (Pearlitol SD 200) | 54.000 |
| Lactose monohydrate *Ph.Eur.* (Flowlac 90) | 134.980 |
| Sodium starch glycolate Type-A *Ph.Eur.* (Explotab) | 24.000 |

| **Stage-D (Blending & Lubrication)** | |
|---|---|
| Magnesium stearate *Ph.Eur.* | 3.500 |
| **Core Tablet weight (mg)** | **348.000** |

| **Stage-E (Film-coating)** | |
|---|---|
| Opadry® Brown *In-house* | 15.000 |
| Water, Purified *Ph.Eur.* | q.s. |
| **Film-coated tablet weight (mg)** | **363.000** |

**Example F33:** inventive pharmaceutical film-coated tablet composition comprising lactose monohydrate, microcrystalline cellulose, sodium starch glycolate, croscarmellose sodium and magnesium stearate in extragranular phase and 96.25 mg eltrombopag olamine (equivalent to 75 mg eltrombopag), microcrystalline cellulose, and polyvinyl pyrrolidone (povidone) in intragranular phase

| **Ingredients** | **F33** |
|---|---|
| **Stage-A: (Dry Mix)** | **(mg/unit)** |
| Eltrombopag Olamine, *In-house* | 96.251 |
| Cellulose, Microcrystalline *Ph.Eur.* (Comprecel M 102 D+) | 18.269 |
| Sodium starch glycolate Type-A *Ph.Eur.* (Explotab) | 7.000 |

| **Stage-B: (Binder Solution / Granulation)** | |
|---|---|
| Polyvinyl pyrrolidone, Povidone *Ph.Eur.* (Plasdone K29/32) | 10.000 |
| Water, purified *Ph.Eur.* | q.s. |

| **Stage-C (Blending)** | |
|---|---|
| Cellulose, Microcrystalline *Ph.Eur.* (Comprecel M 102 D+) | 54.000 |
| Lactose monohydrate *Ph.Eur.* (Flowlac 90) | 120.980 |
| Sodium starch glycolate Type-A *Ph.Eur.* (Explotab) | 28.000 |
| Croscarmellose sodium *Ph.Eur.* (Ac-Di-Sol) | 10.000 |

| **Stage-D (Blending & Lubrication)** | |
|---|---|
| Magnesium stearate *Ph.Eur.* | 3.500 |
| **Core Tablet weight (mg)** | **348.000** |

| **Stage-E (Film-coating)** | |
|---|---|
| Opadry® Brown *In-house* | 15.000 |
| Water, Purified *Ph.Eur.* | q.s. |
| **Film-coated tablet weight (mg)** | **363.000** |

### Part C: Stability and dissolution of inventive pharmaceutical tablet compositions

**Table 1. ASAP Stability data of Coated Tablets, Open Exposure:**

| **Condition** | **Days** | **% Unspecified impurity** | | **% Total impurities** | |
|---|---|---|---|---|---|
| **#** | | **F9** | **F11** | **F9** | **F11** |
| Initial | - | BDL | BDL | BDL | BDL |
| 50°C/40%RH | 14 Days | BDL | BDL | BDL | BDL |
| 50°C/75%RH | 14 Days | 1.79 | 2.29 | 1.98 | 2.49 |
| 60°C/40%RH | 14 Days | BDL | BDL | BDL | BDL |
| 70°C/5%RH | 14 Days | 0.05 | 0.05 | 0.05 | 0.05 |
| 70°C/75%RH | 3 Days | 12.85 | 14.23 | 14.76 | 17.31 |
| 70°C/40%RH | 3 Days | BDL | BDL | BDL | BDL |

| **#** | | **F8** | **F10** | **F8** | **F10** |
|---|---|---|---|---|---|
| Initial | - | BDL | BDL | BDL | BDL |
| 50°C/75%RH | 21 Days | 1.44 | 1.71 | 1.53 | 1.71 |
| 60°C/40%RH | 21 Days | 0.22 | 0.65 | 0.22 | 0.83 |

| **#** | | **F25** | **F26** | **F25** | **F26** |
|---|---|---|---|---|---|
| Initial | - | BDL | BDL | BDL | BDL |
| 50°C/75%RH | 14 Days | 0.60 | BDL | 0.94 | BDL |
| 60°C/40%RH | 14 Days | 0.52 | 0.46 | 0.76 | 0.81 |
| 70°C/5%RH | 14 Days | 0.09 | 0.37 | 0.19 | 0.46 |

| **#** | | **F29** | **F31** | **F29** | **F31** |
|---|---|---|---|---|---|
| Initial | - | BDL | BDL | BDL | BDL |
| 50°C/75%RH | 14 Days | 0.53 | 0.77 | 0.94 | 1.65 |
| 60°C/40%RH | 14 Days | 0.45 | 0.60 | 0.70 | 0.86 |
| 70°C/5%RH | 14 Days | 0.19 | 0.24 | 0.39 | 0.54 |

The amount of impurities measured for the inventive pharmaceutical tablet compositions initially after production are below detection limit (BDL). The impurities measured in the open exposure testing were found to be higher the higher the humidity was. At lower humidity impurities have not been detected even at higher temperatures.

The above data already suggests that inventive eltrombopag olamine tablets packaged preferably in Alu-Alu blister, will show less impurities at real time storage condition, as the water vapor transmission rate (WVTR) for Alu-Alu blister is reduced to the minimum. The finding is confirmed with the accelerated stability testings shown in table 2 below.

Accordingly, under open storage conditions, which are also used in the Kapsi declaration, the degradation of eltrombopag olamine as active ingredient in the inventive pharmaceutical tablet compositions is decreased in comparison to the lactose containing tablet composition of the prior art (see Kapsi declaration), where the tablet composition already initially after production comprises 1.5 % total impurities.

**Table 2. Accelerated stability data of film-coated inventive tablets in Alu-Alu blister:**

| | | | | **40 °C / 75 % RH** | | | **50°C / 75 % RH** |
|---|---|---|---|---|---|---|---|
| **Composition** | **Specification** | **Impurities** | **Initial** | **1 month** | **2 month** | **6 month** | **15 days** |
| **F5** | Not more than 0.2 | **Unknown** | BDL | BDL | BDL | | |
| | Not more than 2.0 | **Total** | BDL | BDL | BDL | | |
| **F7** | Not more than 0.2 | **Unknown** | BDL | BDL | BDL | | |
| | Not more than 2.0 | **Total** | BDL | BDL | BDL | | |
| **F8** | Not more than 0.2 | **Unknown** | BDL | BDL | BDL | | |
| | Not more than 2.0 | **Total** | BDL | BDL | BDL | | |
| **F9** | Not more than 0.2 | **Unknown** | BDL | BDL | BDL | BDL | BDL |
| | Not more than 2.0 | **Total** | BDL | BDL | BDL | BDL | BDL |
| **F10** | Not more than 0.2 | **Unknown** | BDL | BDL | BDL | | |
| | Not more than 2.0 | **Total** | BDL | BDL | BDL | | |
| **F11** | Not more than 0.2 | **Unknown** | BDL | BDL | BDL | BDL | BDL |
| | Not more than 2.0 | **Total** | BDL | BDL | BDL | BDL | BDL |

The physical and chemical stability of the inventive pharmaceutical tablet composition may be tested in conventional manner, in particular at accelerated conditions according to ICH guideline Q1A(R2) (i.e. at 25 °C / 60 % relative humidity (RH) and/or at 40 °C / 75 % RH). The accelerated stability tests as set out in table 2 above have been performed according to applicable pharmaceutical regulatory standards as described e.g. in ICH or EMA guidelines and/or the European Pharmacopeia (EP), wherein the inventive pharmaceutical tablet composition have been packed in Alu-Alu blisters.

The amount of impurities measured for the inventive pharmaceutical tablet compositions initially after production are below detection limit. When using the regulatory accelerated stability assessment standards for Europe as storage conditions, the inventive pharmaceutical tablet compositions are sealed in Alu-Alu blisters and the amount of impurities is still below detection limit after one or two or six months storage. Accordingly, the inventive pharmaceutical tablet compositions show no degradation within two month accelerated storage.

The fact that no degradation products of eltrombopag olamine could be detected may be due to the separation of eltrombopag olamine in the intragranular phase and the reducing sugar, preferably lactose in the extragranular phase. When using higher amounts of reducing sugar, in particular lactose, the additional use of a polymeric binder agent, in particular povidone, in the intragranular phase and/or extragranular phase, in particular the extragranular phase and optionally the intragranular phase seems to be advantageous to further facilitate the decreased degradation of eltrombopag olamine in the inventive tablet composition.

**Table 3. Dissolution testing:**

| **Composition** | **Cumulative wt.% eltrombopag olamine after 45 minutes** |
|---|---|
| **F1** | **97** |
| **F3** | **96** |
| **F4** | **87** |
| **F9** | **94** |
| **F11** | **96** |

Dissolution testing according to tables 3-7 was performed using USP Apparatus II, 50 rpm, 900 ml of phosphate buffer pH 6.8 containing 0.5% Tween 80.

The experimental data of table 3 shows that 75 wt.% or more, preferably more than 80 wt.%, more preferably more than 90 wt.% of eltrombopag olamine of the inventive pharmaceutical tablet compositions is dissolved within 45 minutes in accordance with the regulatory standard test.

With respect to inventive eltrombopag tablet compositions F9 and F11 the test results could be confirmed after 1 months storage under accelerated condition at 40°C and 75 % relative humidity. Accordingly, the inventive tablet compositions are not only stable over preferably 6 month, they also comply with the regulatory standards for dissolution testings, where at least 75 wt.% of eltrombopag olamine needs to be dissolved within 45 minutes.

In addition dissolution testings concerning, e.g. F17, F18, F20, F21, F23, and F25-F33, show that after 45 minutes at least 75 wt.%, preferably between 86 and 100 wt.% eltrombopag olamine based on the total weight of the eltrombopag olamine content in the tablet is dissolved.

Furthermore, the dissolution of the inventive formulations of the present invention is comparable to the commercially available reference product Revolade® film-coated tablets (FCTs), as shown below in tables 4, 5, 6, and 7 for the 75 mg, 50 mg, 25 mg, and 12.5 mg strength, correspondingly.

**Table 4. Comparative dissolution of Eltrombopag 75 mg FCTs with Revolade® 75 mg:**

| **Composition** | **Cumulative wt.% eltrombopag olamine dissolved after 45 minutes** |
|---|---|
| **Revolade® 75 mg (batch MX4H)** | **97** |
| **F25** | **93** |
| **F32** | **94** |
| **F33** | **95** |

**Table 5. Comparative dissolution of Eltrombopag 50 mg FCTs with Revolade® 50 mg:**

| **Composition** | **Cumulative wt.% eltrombopag olamine dissolved after 45 minutes** |
|---|---|
| **Revolade® 50 mg (batch 6Y6C)** | **94** |
| **F17** | **93** |
| **F26** | **99** |
| **F29** | **92** |

**Table 6. Comparative dissolution of Eltrombopag 25 mg FCTs with Revolade® 25 mg:**

| **Composition** | **Cumulative wt.% eltrombopag olamine dissolved after 45 minutes** |
|---|---|
| **Revolade® 25 mg (batch 3B9C)** | **86** |
| **F18** | **91** |
| **F27** | **94** |
| **F30** | **94** |

**Table 7. Comparative dissolution of Eltrombopag 12.5 mg FCTs with Revolade® 12.5 mg:**

| **Composition** | **Cumulative wt.% eltrombopag olamine dissolved after 45 minutes** |
|---|---|
| **Revolade® 12.5 mg (batch 9Y3C)** | **87** |
| **F20** | **88** |
| **F21** | **94** |
| **F23** | **92** |
| **F28** | **93** |
| **F31** | **91** |

## Claims

1. Pharmaceutical tablet composition comprising eltrombopag olamine as active ingredient and one or more pharmaceutically acceptable excipients including one or more reducing sugars, **characterized in that** the composition comprises or consists of
a) eltrombopag olamine in a therapeutically effective amount and one or more pharmaceutically acceptable excipients in an intragranular composition, and
b) one or more reducing sugars comprising lactose and optionally further one or more pharmaceutically acceptable excipients in an extragranular composition.

2. Pharmaceutical tablet composition according to claim 1, wherein the reducing sugar in the extragranular composition comprises or consists of lactose selected from the group consisting of lactose monohydrate, anhydrous lactose, spray dried lactose and co-processed lactose.

3. Pharmaceutical tablet composition according to claim 1 or 2, wherein the reducing sugar content is greater than 5 wt.%, preferably at least 9 wt.%, more preferably in the range of 15 to 75 wt.% respectively based on the total weight of the pharmaceutical tablet composition.

4. Pharmaceutical tablet composition according to any one of claims 1 to 3, wherein the further one or more pharmaceutically acceptable excipients in the intragranular and optionally extragranular composition are selected from suitable binder agents, preferably one or more binder agents are selected from the group consisting of povidone, copovidone, hydroxypropyl cellulose, low-substituted hydroxypropyl cellulose, hydroxypropyl methylcellulose, hydroxypropyl starch, polyethylene oxide, more preferably one or two binder agents are selected from povidone and copovidone.

5. Pharmaceutical tablet composition according to any one of claims 1 to 4, wherein the further one or more pharmaceutically acceptable excipients in the intragranular and/or extragranular composition, preferably in the intragranular and extragranular composition are selected from suitable further diluent agents, preferably one or more further diluent agents are selected from the group consisting of erythritol, isomalt, maltitol, xylitol, microcrystalline cellulose, powdered cellulose, pregelatinized starch, starch, lactitol, mannitol, sorbitol, maltodextrin more preferably one, two or more further diluent agents are selected from erythritol, isomalt, maltitol, xylitol, and microcrystalline cellulose, even more preferably the further diluent agents are selected from microcrystalline cellulose and one, two or more selected from erythritol, isomalt, maltitol and xylitol, preferably xylitol.

6. Pharmaceutical tablet composition according to any one of claims 1 to 5 wherein the further one or more pharmaceutically acceptable excipients in the intragranular and/or extragranular composition, preferably in the extragranular composition are selected from suitable disintegrant agents, preferably one or more disintegrant agents are selected from the group consisting of sodium starch glycolate, crospovidone, and croscarmellose sodium, more preferably one or two selected from sodium starch glycolate and crospovidone.

7. Pharmaceutical tablet composition according to any one of claims 1 to 6, wherein the further one or more pharmaceutically acceptable excipients in the extragranular composition are selected from suitable lubricant agents, preferably selected from magnesium stearate, stearic acid, and sodium stearyl fumarate, more preferably magnesium stearate.

8. Pharmaceutical tablet composition according to any one of claims 1 to 7, wherein the pharmaceutical tablet composition is film coated with a suitable coating agent, preferably comprising one or more pharmaceutically acceptable polymers, optionally one or more pharmaceutically acceptable plasticizers, and optionally one or more pharmaceutically acceptable pigments.

9. Pharmaceutical tablet composition according to any one of claims 4 to 8, wherein
i. the total content of binder agent(s) is at least 0.5 wt.% or more, more preferably is in the range of 0.5 to 7 wt.%, and/or
ii. the total content of diluent agent(s) including the reducing sugar amount is at least 5 wt.% or more, preferably is present in the range of 15 to 80 wt.%, preferably 7 to 62 wt.%, and/or
iii. the total content of the disintegrant agent(s) is at least 3 wt.% or more, preferably is in the range of 4 to 15 wt.%, more preferably 5 to 14 wt.%, even more preferably 6 to 12 wt.% and/or
iv. the total content of the lubricant agent(s) is at least 0.5 wt.% or more, preferable is present in the range of 0.5 to 5 wt.%, preferably 0.8 to 3 wt.%, more preferably 0.9 to 1.5 wt.% and/or
v. the total content of the coating agent(s) is at least 2 wt.%, preferably in the range of 3 to 7 wt.%, more preferably 4 to 5 wt.%,
respectively based on the total weight of the pharmaceutical tablet composition.

10. Pharmaceutical tablet composition according to any one of the preceding claims for use in the treatment or prophylaxis of immune (idiopathic) thrombocytopenic purpura (ITP), thrombocytopenia and/or acquired severe aplastic anaemia (SAA).

11. Process of production of a pharmaceutical tablet comprising eltrombopag olamine as active ingredient and one or more pharmaceutically acceptable excipients including one or more reducing sugars, **characterized in that** the process comprises the following steps:
a. mixing a therapeutically effective amount of eltrombopag olamine and one or more pharmaceutically acceptable excipients,
b. wet granulation of components mixed in step a) with a suitable granulation fluid, preferably water or an aqueous solution of a binder, in a suitable amount to form an intragranular composition,
c. mixing one or more reducing sugars comprising lactose and optionally further one or more pharmaceutically acceptable excipients to form an extragranular composition,
d. mixing the extragranular composition of step c) with the intragranular composition of step b) to form a tablet composition, and
e. compressing the tablet composition of step d) to form the pharmaceutical tablet composition comprising eltrombopag olamine as active ingredient in the intragranular phase and one or more reducing sugars comprising lactose in the extragranular phase.

12. Process according to claim 12, wherein the compressed pharmaceutical tablet composition of step d) is in a subsequent step e) film coated with one or more pharmaceutically acceptable excipients.

13. Pharmaceutical tablet composition comprising eltrombopag olamine as active ingredient and one or more pharmaceutically acceptable excipients including one or more reducing sugars, **characterized in that** the composition comprising or consisting of
a) eltrombopag olamine in a therapeutically effective amount and one or more pharmaceutically acceptable excipients in an intragranular composition, and
b) one or more reducing sugars comprising lactose and optionally further one or more pharmaceutically acceptable excipients in an extragranular composition
is obtainable by the production process according to claim 11 or 12.

## Patentansprüche

1. Pharmazeutische Tablettenzusammensetzung umfassend Eltrombopag-Olamin als Wirkstoff und einen oder mehrere pharmazeutisch akzeptable Hilfsstoffe einschließlich eines oder mehrere reduzierender Zucker, **dadurch gekennzeichnet, dass** die Zusammensetzung umfasst oder besteht aus
a) Eltrombopag-Olamin in einer therapeutisch wirksamen Menge und einem oder mehreren pharmazeutisch akzeptablen Hilfsstoffe in einer intragranularen Zusammensetzung und
b) einem oder mehreren reduzierenden Zucker, umfassend Lactose und gegebenenfalls einen oder mehrere weitere pharmazeutisch akzeptable Hilfsstoffe in einer extragranularen Zusammensetzung.

2. Pharmazeutische Tablettenzusammensetzung gemäß Anspruch 1, wobei der reduzierende Zucker in der extragranularen Zusammensetzung umfasst oder besteht aus Lactose, ausgewählt aus der Gruppe bestehend aus Lactose-Monohydrat, wasserfreie Lactose, sprühgetrocknete Lactose oder coprozessierte Lactose.

3. Pharmazeutische Tablettenzusammensetzung gemäß Anspruch 1 oder 2, wobei der reduzierende Zuckeranteil größer als 5 Gew.-% ist, vorzugsweise zumindest 9 Gew.-%, bevorzugter im Bereich zwischen 15 bis 75 Gew.-%, jeweils bezogen auf das Gesamtgewicht der pharmazeutischen Zusammensetzung.

4. Pharmazeutische Tablettenzusammensetzung gemäß einem der Ansprüche 1 bis 3, wobei der eine oder die mehreren weiteren pharmazeutisch akzeptablen Hilfsstoffe in der intragranularen und gegebenenfalls extragranularen Zusammensetzung ausgewählt sind aus geeigneten Bindemitteln, vorzugsweise einem oder mehreren Bindemitteln ausgewählt aus der Gruppe bestehend aus Povidon, Copovidon, Hydroxypropylcellulose, niedrigsubstituierter Hydroxypropylcellulose, Hydroxypropylmethylcellulose, Hydroxypropylstärke, Polyethylenoxid, bevorzugter einem oder zwei Bindemitteln ausgewählt aus Povidon and Copovidon.

5. Pharmazeutische Tablettenzusammensetzung gemäß einem der Ansprüche 1 bis 4, wobei der eine oder die mehreren weiteren pharmazeutisch akzeptablen Hilfsstoffe in der intragranularen und/oder extragranularen Zusammensetzung, vorzugsweise in der intragranularen und extragranularen Zusammensetzung ausgewählt sind aus geeigneten weiteren Füllstoffen, vorzugsweise einem oder mehreren weiteren Füllstoffen ausgewählt aus der Gruppe bestehend aus Erythritol, Isomalt, Maltitol, Xylitol, mikrokristalliner Cellulose, Cellulosepulver, modifizierter Stärke, Stärke, Lactitol, Mannitol, Sorbitol, Maltodextrin, bevorzugter einem oder mehreren weiteren Füllstoffen ausgewählt aus Erythritol, Isomalt, Maltitol, Xylitol und mikrokristalliner Cellulose, noch bevorzugter sind die weiteren Füllstoffe ausgewählt aus mikrokristalliner Cellulose und ein, zwei oder mehreren ausgewählt aus Erythritol, Isomalt, Maltitol und Xylitol, vorzugsweise Xylitol.

6. Pharmazeutische Tablettenzusammensetzung gemäß einem der Ansprüche 1 bis 5, wobei der eine oder die mehreren weiteren pharmazeutisch akzeptablen Hilfsstoffe in der intragranularen und/oder extragranularen Zusammensetzung, vorzugsweise in der extragranularen Zusammensetzung ausgewählt sind aus geeigneten Sprengmitteln, vorzugsweise einem oder mehreren Sprengmitteln ausgewählt aus der Gruppe bestehend aus Natrium-Stärke-Glykolat, Crospovidon, Croscarmellose-Natrium, bevorzugter ein oder zwei ausgewählt aus Natrium-Stärke-Glykolat und Crospovidon.

7. Pharmazeutische Tablettenzusammensetzung gemäß einem der Ansprüche 1 bis 6, wobei der eine oder die mehreren weiteren pharmazeutisch akzeptablen Hilfsstoffe in der extragranularen Zusammensetzung ausgewählt sind aus geeigneten Schmiermitteln, vorzugsweise ausgewählt aus Magnesiumstearat, Stearinsäure und Natriumstearylfumarat, bevorzugter Magnesiumstearat.

8. Pharmazeutische Tablettenzusammensetzung gemäß einem der Ansprüche 1 bis 7, wobei die pharmazeutische Zusammensetzung filmbeschichtet ist mit einem geeigneten Filmüberszugsmitttel, vorzugsweise umfassend ein oder mehrere pharmazeutisch akzeptable Polymere, gegebenenfalls einen oder mehrere pharmazeutisch akzeptable Weichmacher und gegebenenfalls ein oder mehrere pharmazeutisch akzeptable Pigmente.

9. Pharmazeutische Tablettenzusammensetzung gemäß einem der Ansprüche 4 bis 8, wobei
i. der Gesamtanteil des/der Bindemittel(s) zumindest 0,5 Gew.-% oder mehr beträgt, bevorzugter in dem Bereich von 0,5 bis 7 Gew.-% liegt, und/oder
ii. der Gesamtanteil des/der Füllstoffe/s einschließlich des reduzierenden Zuckeranteils zumindest 5 Gew.-% oder mehr beträgt, vorzugsweise im Bereich von 15 bis 80 Gew.-% liegt, vorzugsweise 7 bis 62 Gew.-%, und/oder
iii. der Gesamtanteil des/der Sprengmittel(s) zumindest 3 Gew.-% oder mehr beträgt, vorzugsweise im Bereich von 4 bis 15 Gew.-% liegt, bevorzugter 5 bis 14 Gew.-%, noch bevorzugter 6 bis 12 Gew.-%, und/oder
iv. der Gesamtanteil des/der Schmiermittel(s) zumindest 0,5 Gew.-% oder mehr beträgt, vorzugsweise im Bereich von 0,5 bis 5 Gew.-% liegt, vorzugsweise 0,8 bis 3 Gew.-%, bevorzugter 0,9 bis 1,5 Gew.-%, und/oder
v. der Gesamtanteil des/der Filmüberzugsmittel(s) zumindest 2 Gew.-% beträgt, vorzugsweise im Bereich von 3 bis 7 Gew.-% liegt, bevorzugter 4 bis 5 Gew.-%,
jeweils bezogen auf das Gesamtgewicht der pharmazeutischen Tablettenzusammensetzung.

10. Pharmazeutische Tablettenzusammensetzung gemäß einem der vorstehenden Ansprüche zur Verwendung in der Behandlung oder Prophylaxe von immun (idiopathischer) thrombozytopenischer Purpura (ITP), Thrombozytopenie und/oder erworbener schwerer aplastischer Anämie (SAA).

11. Verfahren zur Herstellung einer pharmazeutischen Tablette umfassend Eltrombopag-Olamin als Wirkstoff und einen oder mehrere pharmazeutisch akzeptable Hilfsstoffe einschließlich eines oder mehrerer reduzierender Zucker, **dadurch gekennzeichnet, dass** das Verfahren die folgenden Schritte umfasst oder daraus besteht:
a. mischen einer therapeutisch wirksamen Menge Eltrombopag-Olamin und eines oder mehrerer pharmazeutisch akzeptablen Hilfsstoffe,
b. naßgranulieren von Bestandteilen, die in Schritt a) gemischt wurden, mit einer geeigneten Granulierflüssigkeit, vorzugsweise Wasser oder einer wässrigen Lösung eines Bindemittels, in einer geeigneten Menge, um eine intragranulare Zusammensetzung zu bilden,
c. mischen von einem oder mehreren reduzierenden Zuckern umfassend Lactose und gegebenenfalls einen oder mehrere weitere pharmazeutisch akzeptablen Hilfsstoffe, um eine extragranulare Zusammensetzung zu bilden,
d. mischen der extragranularen Zusammensetzung aus Schritt c) mit der intragranularen Zusammensetzung aus Schritt b), um eine Tablettenzusammensetzung zu bilden, und
e. verpressen der Tablettenzusammensetzung aus Schritt d), um die pharmazeutische Tablettenzusammensetzung umfassend Eltrombopag-Olamin als Wirkstoff in der intragranularen Phase und einen oder mehrere reduzierende Zucker umfassend Lactose in der extragranularen Phase zu bilden.

12. Verfahren gemäß Anspruch 12, wobei die verpresste pharmazeutische Tablettenzusammensetzung aus Schritt d) in einem anschließenden Schritt e) mit einem oder mehreren pharmazeutisch akzeptablen Hilfsstoffen filmbeschichtet wird.

13. Pharmazeutische Tablettenzusammensetzung umfassend Eltrombopag-Olamin als Wirkstoff und einen oder mehrere pharmazeutisch akzeptable Hilfsstoffe einschließlich eines oder mehrerer reduzierender Zucker, **dadurch gekennzeichnet, dass** die Zusammensetzung umfassend oder bestehend aus:
a) Eltrombopag-Olamin in einer therapeutisch wirksamen Menge und einem oder mehreren pharmazeutisch akzeptablen Hilfsstoffen in einer intragranularen Zusammensetzung, und
b) einem oder mehreren reduzierenden Zuckern, umfassend Lactose und gegebenenfalls einen oder mehrere weitere pharmazeutisch akzeptablen Hilfsstoffe, in einer extragranularen Zusammensetzung,
nach dem Verfahren gemäß Anspruch 11 oder 12 erhältlich ist.

## Revendications

1. Composition de comprimé pharmaceutique comprenant de l'eltrombopag olamine comme ingrédient actif et un ou plusieurs excipients pharmaceutiquement acceptables comprenant un ou plusieurs sucres réducteurs, **caractérisée en ce que** la composition comprend ou consiste en
a) de l'eltrombopag olamine en une quantité thérapeutiquement efficace et un ou plusieurs excipients pharmaceutiquement acceptables dans une composition intragranulaire, et
b) un ou plusieurs sucres réducteurs comprenant du lactose et, facultativement, un ou plusieurs excipients supplémentaires pharmaceutiquement acceptables dans une composition extragranulaire.

2. Composition de comprimé pharmaceutique selon la revendication 1, dans laquelle le sucre réducteur dans la composition extragranulaire comprend ou consiste en du lactose choisi dans le groupe consistant en du lactose monohydraté, du lactose anhydre, du lactose séché par pulvérisation et du lactose co-traité.

3. Composition de comprimé pharmaceutique selon la revendication 1 ou 2, dans laquelle le taux de sucre réducteur est supérieur à 5% en poids, de préférence à au moins 9% en poids, encore plus préférablement dans la gamme de 15 à 75% en poids, par raport au poids total de la composition de comprimé pharmaceutique.

4. Composition de comprimé pharmaceutique selon l'une quelconque des revendications 1 à 3, dans laquelle les un ou plusieurs excipients supplémentaires pharmaceutiquement acceptables dans la composition intragranulaire, et facultativement extragranulaire, sont choisis parmi des agents liants appropriés, de préférence un ou plusieurs agents liants sont choisis dans le groupe consistant en : povidone, copovidone, hydroxypropylcellulose, hydroxypropylcellulose faiblement substituée, hydroxypropylméthylcellulose, amidon hydroxypropylé, oxyde de polyéthylène, plus préférablement un ou deux agents liants sont choisis parmi la povidone et la copovidone.

5. Composition de comprimé pharmaceutique selon l'une quelconque des revendications 1 à 4, dans laquelle les un ou plusieurs excipients supplémentaires pharmaceutiquement acceptables dans la composition intragranulaire et/ou extragranulaire, de préférence dans la composition intragranulaire et extragranulaire, sont choisis parmi des agents diluants supplémentaires appropriés, de préférence un ou plusieurs agents diluants supplémentaires sont choisis dans le groupe consistant en: érythritol, isomalt, maltitol, xylitol, cellulose microcristalline, cellulose en poudre, amidon prégélatinisé, amidon, lactitol, mannitol, sorbitol, maltodextrine; plus préférablement un, deux ou plusieurs agents diluants supplémentaires sont choisis parmi l'érythritol, l'isomalt, le maltitol, le xylitol et la cellulose microcristalline; encore plus préférablement, les agents diluants supplémentaires sont choisis parmi la cellulose microcristalline et un, deux ou plusieurs sont choisis parmi l'érythritol, l'isomalt, le maltitol et le xylitol, de préférence le xylitol.

6. Composition de comprimé pharmaceutique selon l'une quelconque des revendications 1 à 5, dans laquelle les un ou plusieurs excipients supplémentaires pharmaceutiquement acceptables dans la composition intragranulaire et/ou extragranulaire, de préférence dans la composition extragranulaire, sont choisis parmi des agents désintégrants appropriés, de préférence un ou plusieurs agents désintégrants sont choisis dans le groupe consistant en: glycolate d'amidon sodique, crospovidone et sodium de croscarmellose, plus préférablement un ou deux sont choisis parmi le glycolate d'amidon sodique et la crospovidone.

7. Composition de comprimé pharmaceutique selon l'une quelconque des revendications 1 à 6, dans laquelle les un ou plusieurs excipients supplémentaires pharmaceutiquement acceptables dans la composition extragranulaire, sont choisis parmi des agents lubrifiants appropriés, de préférence choisis parmi le stéarate de magnésium, l'acide stéarique et le stéaryl-fumarate de sodium, plus préférablement le stéarate de magnésium.

8. Composition de comprimé pharmaceutique selon l'une quelconque des revendications 1 à 7, dans laquelle la composition de comprimé pharmaceutique est pelliculée avec un agent d'enrobage approprié, comprenant de préférence un ou plusieurs polymères pharmaceutiquement acceptables, facultativement un ou plusieurs plastifiants pharmaceutiquement acceptables, et facultativement un ou plusieurs pigments pharmaceutiquement acceptables.

9. Composition de comprimé pharmaceutique selon l'une quelconque des revendications 4 à 8, dans laquelle
i. la teneur totale en agent(s) liant(s) est au moins de 0,5% en poids ou plus, plus préférablement dans la gamme de 0,5 à 7% en poids, et/ou
ii. la teneur totale en agent(s) diluant(s), y compris la quantité de sucre réducteur, est au moins de 5% en poids ou plus, préférablement se trouve dans la gamme de 15 à 80% en poids, préférablement de 7 à 62 % en poids, et/ou
iii. la teneur totale en agent(s) désintégrant(s) est au moins de 3% en poids ou plus, préférablement est dans la gamme de 4 à 15% en poids, plus préférablement de 5 à 14% en poids, encore plus préférablement de 6 à 12% en poids, et/ou
iv. la teneur totale en agent(s) lubrifiant(s) est au moins de 0,5% en poids ou plus, préférablement se trouve dans la gamme de 0,5 à 5% en poids, préférablement de 0,8 à 3% en poids, encore plus préférablement de 0,9 à 1,5% en poids, et/ou
v. la teneur totale en agent(s) d'enrobage est au moins de 2% en poids, préférablement dans la gamme de 3 à 7% en poids, plus préférablement de 4 à 5% en poids,
par raport au poids total de la composition de comprimé pharmaceutique.

10. Composition de comprimé pharmaceutique selon l'une quelconque des revendications précédentes pour utilisation pour le traitement ou la prévention de la purpura thrombopénique immunologique (idiopathique) (PTI), la thrombocytopénie et/ou l'aplasie médullaire acquise sévère (AMS).

11. Procédé de production d'un comprimé pharmaceutique comprenant de l'eltrombopag olamine comme ingrédient actif et un ou plusieurs excipients pharmaceutiquement acceptables comprenant un ou plusieurs sucres réducteurs, **caractérisé en ce que** le procédé comprend les étapes suivantes :
a. mélanger une quantité thérapeutiquement efficace d'eltrombopag olamine avec un ou plusieurs excipients pharmaceutiquement acceptables,
b. granulation humide de composants mélangés à l'étape a) avec un fluide de granulation approprié, de préférence de l'eau ou une solution aqueuse d'un liant, en quantité appropriée, pour former une composition intragranulaire,
c. mélanger un ou plusieurs sucres réducteurs comprenant du lactose et, facultativement, un ou plusieurs excipients supplémentaires pharmaceutiquement acceptables pour former une composition extragranulaire,
d. mélanger la composition extragranulaire de l'étape c) avec la composition intragranulaire de l'étape b) pour former une composition de comprimé, et
e. comprimer la composition de comprimé de l'étape d) pour former la composition de comprimé pharmaceutique comprenant de l'eltrombopag olamine comme ingrédient actif dans la phase intragranulaire et un ou plusieurs sucres réducteurs comprenant du lactose dans la phase extragranulaire.

12. Procédé selon la revendication 12, dans lequel la composition de comprimé pharmaceutique comprimé de l'étape d) est pelliculée avec un ou plusieurs excipients pharmaceutiquement acceptables lors d'une étape ultérieure e).

13. Composition de comprimé pharmaceutique comprenant de l'eltrombopag olamine comme ingrédient actif et un ou plusieurs excipients pharmaceutiquement acceptables comprenant un ou plusieurs sucres réducteurs, **caractérisé en ce que** la composition comprenant ou consistant en
a) de l'eltrombopag olamine en une quantité thérapeutiquement efficace et un ou plusieurs excipients pharmaceutiquement acceptables dans une composition intragranulaire, et
b) un ou plusieurs sucres réducteurs comprenant du lactose et, facultativement, un ou plusieurs excipients supplémentaires pharmaceutiquement acceptables dans une composition extragranulaire,
peut être obtenue par le procédé de production selon la revendication 11 ou 12.
